# EUROPEAN PATENT APPLICATION

(11) **EP 4 428 123 A1**
(43) Date of publication of application: **11.09.2024**
(21) Application number: 22884989.9
(22) Date of filing: 15.04.2022
(51) Int. Cl.: C07D 235/18, C07D 413/12, C07D 417/12, C07D 403/12, C07D 269/02, C07D 277/62, H10K 50/00, H10K 85/00

(54) **ARYLAMINE ORGANIC COMPOUND AND USE THEREOF**

(30) Priority: 01.11.2021 CN 202111283818
(71) Applicant: Guangzhou Chinaray Optoelectronic Materials Ltd., Guangzhou, Guangdong 510663 (CN)
(72) Inventor: LI, Tao, Guangzhou, Guangdong 510663 (CN); LONG, Zhifei, Guangzhou, Guangdong 510663 (CN); SONG, Jingyao, Guangzhou, Guangdong 510663 (CN)
(74) Representative: Nederlandsch Octrooibureau
(86) International application number: PCT/CN2022/087065
(87) International publication number: WO 2023/071073

(57) **Abstract**

The present application discloses an arylamine organic compound and the use thereof. The arylamine organic compound has a structure as shown in general formula (1). 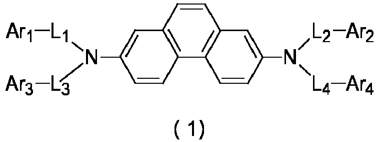 The arylamine organic compound has a relatively high glass transition temperature, a relatively high thermal stability, a relatively small visible light extinction coefficient, and a relatively high refractive index; and when the arylamine organic compound is used as a capping layer material for an organic electronic device, the visible luminous efficiency of the organic electronic device can be effectively improved.

## Description

### TECHNICAL FIELD

The present application relates to the technical field of light-emitting materials, and more particularly, to an arylamine organic compound, a mixture including the arylamine organic compound, and an organic electronic device.

### BACKGROUND

Organic light-emitting display device is a kind of self-luminous display device, which generates excitons through the transfer and recombination of carriers between functional layers, and emits light by organic compounds or metal complexes with high quantum efficiency, and has the characteristics of self-luminescence, high brightness, high efficiency, high contrast and high responsiveness.

In recent years, the luminous efficiency of organic light-emitting diodes (OLEDs) has been greatly improved, but the internal quantum efficiency is close to the theoretical limit. As for the organic light-emitting diode, when light emitted by the light-emitting layer is incident on other films, if it is incident at a certain angle or more angles, total reflection will occur at the interface between the light-emitting layer and other films, as a result, light emitted by the light-emitting layer cannot be exited out of the device, so that only a part of the light emitted from the light-emitting layer can be utilized. Therefore, improving the light extraction efficiency has become an effective means to further improve device stability and current efficiency.

In recent years, in order to improve the light extraction efficiency, a light-emitting device in which a "covering layer" (light extraction layer) with a high refractive index is provided outside a translucent electrode with a low refractive index has been proposed. For example, in 2001, Hung et al. coated the surface of the metal cathode with a layer of organic compounds or inorganic compounds of about 50 nm to improve the performance of the device by controlling the thickness and refractive index. In 2003, Riel et al., tried to evaporate an inorganic compound ZnSe with a high refractive index (n= 2.6) on a cathode to improve light extraction efficiency by using the difference in refractive indices between functional layers. However, such compounds have not been widely used in organic electronic devices due to reasons such as high evaporation temperature and slow evaporation rate of inorganic materials.

Therefore, a new class of materials that can improve the light extraction efficiency of organic electronic devices needs to be further developed.

### TECHNICAL PROBLEMS

The luminous efficiency of organic electronic devices in related arts need to be further improved.

### TECHNICAL SOLUTIONS

The present application provides an arylamine organic compound, a mixture including the arylamine organic compound, and an organic electronic device.

The technical solutions of the present application are as follows:

An arylamine organic compound having a structure represented by formula (1): 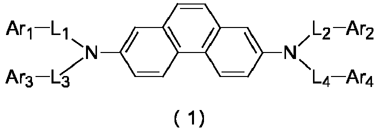
L₁ to L₄ are each independently selected from a single bond, a substituted or unsubstituted aromatic group having 6 to 30 ring atoms, or a substituted or unsubstituted heteroaromatic group having 5 to 30 ring atoms;
Ar₁ is selected from any structure represented by formula (A-1) or formula (A-2);
Ar₂ is selected from any structure represented by formulas (A-2) to (A-6);
Ar₃ and Ar₄ are each independently selected from any structure represented by formulas (A-3) to (A-6) described above;
structures represented by formulas (A-1) to (A-6) are as follows: 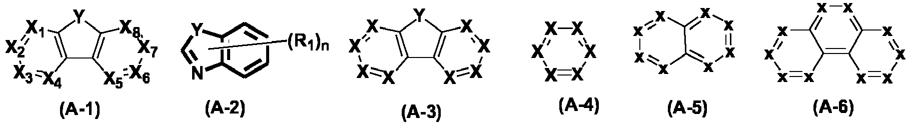
wherein n is selected from 0, 1, 2, 3 or 4;
Y is selected from O, S or NR₂;
X and X₁ to X₈ are each independently selected from CR₃ or N, respectively, and at least one of X₁ to X₈ is selected from N; and
R₁, R₂ and R₃ are each independently selected from -H, -D, a linear alkyl group having 1 to 20 carbon atoms, a linear alkoxy group having 1 to 20 carbon atoms, a branched alkyl group having 3 to 20 carbon atoms, a branched alkoxy group having 3 to 20 carbon atoms, a cyclic alkyl group having 3 to 20 carbon atoms, a cyclic alkoxy group having 3 to 20 carbon atoms, a substituted or unsubstituted silyl group, a cyano group, an isocyano group, a hydroxyl group, a nitro group, an amino group, -CF₃, -Cl, -Br, -F, -I, an alkenyl group having 2 to 20 carbon atoms, or a substituted or unsubstituted aromatic group having 6 to 20 ring atoms, or a combination thereof.

The present application further provides a mixture including an arylamine organic compound and at least one organic functional material. The at least one organic functional material is selected from a hole injection material, a hole transport material, an electron transport material, an electron injection material, an electron blocking material, a hole blocking material, a light-emitting material, a host material, a host material or an organic dye.

The present application further provides an organic electronic device including two electrodes, one or more organic functional layers disposed between the two electrodes, and a light extraction layer disposed on a surface of one of the electrodes and on a side away from the organic functional layer. The light extraction layer includes at least one arylamine organic compound described above.

### BENEFICIAL EFFECTS

The arylamine organic compounds of the present application contain phenanthrene, diarylamine, and fused heterocyclic ring in the structures, and have high glass temperature and high thermal stability. In addition, the arylamine organic compounds have high extinction coefficient for ultraviolet light, and when the arylamine organic compounds are used as a light extraction layer of an organic electronic device, adverse effects of harmful light on electrodes and internal functional layers of the organic electronic device can be effectively avoided. Furthermore, the arylamine organic compound has a relatively small extinction coefficient for visible light and a relatively high transmittance for visible light, thereby reducing the influence on the luminous efficiency of the device. When the arylamine organic compounds are used as light extraction layer materials in an organic electronic device, the luminous efficiency of visible light of the device can be effectively improved.

### BRIEF DESCRIPTION OF THE DRAWINGS

In order to more clearly describe the technical solutions in the embodiments of the present application, hereinafter, the appended drawings used for describing the embodiments will be briefly introduced. Apparently, the appended drawings described below are only directed to some embodiments of the present application, and for a person skilled in the art, other drawings can be derived on the basis of these appended drawings without expenditure of creative labor.
FIG. 1 is a schematic structural diagram of an organic electronic device according to some embodiments of the present application;
FIG. 2 is an ultraviolet-visible absorption spectrum of organic compound C1 of Example 1 in dichloromethane;
FIG. 3 is an ultraviolet-visible absorption spectrum of organic compound C21 of Example 2 in dichloromethane.

### EMBODIMENTS OF THE PRESENT APPLICATION

Hereinafter, technical solutions in embodiments of the present application will be clearly and completely described with reference to the drawings in embodiments of the present application. Obviously, the described embodiments are part of, but not all of, the embodiments of the present application. All the other embodiments, obtained by a person with ordinary skill in the art on the basis of the embodiments in the present application without expenditure of creative labor, belong to the protection scope of the present application. In addition, it should be understood that specific embodiments described herein are only used to illustrate and explain the present application, and are not intended to limit the present application. In the present application, unless otherwise stated, orientation words such as "up" and "down" generally refers to "up" and "down" in the actual use or working state of a device. In addition, in the description of the present application, the term "comprises" means "including but not limited to". The term "plurality" means "two or more". Throughout the present application, various embodiments of the present application may be presented in a range format. It should be understood that the description in range format is merely for convenience and brevity and should not be construed as a hard limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. Whenever a numerical range is indicated herein, it is meant to include any cited numeral (fractional or integral) within the indicated range.

In the present application, aromatic groups, aromatic, aromatic ring systems have the same meaning and are interchangeable.

In the present application, heteroaromatic groups, heteroaromatic, heteroaromatic ring systems have the same meaning and are interchangeable.

In the present application, "substituted" means that the hydrogen atom/hydrogen atoms in a substituent is/are substituted by one or more substituents

In the present application, when the same substituent occurs multiple times, it may be independently selected from different groups, for example, when the formula contains a plurality of R₁, then R₁ may be independently selected from different groups.

In the present application, "substituted or unsubstituted" refers to that the defined group may be substituted or may not be substituted. When the defined group is substituted, it should be understood that it is substituted by one or more substituents R, and R is selected but not limited to, deuterium atom, cyano group, isocyano group, nitro group or halogen, alkyl groups having 1 to 20 carbon atoms, heterocyclyl groups having 3 to 20 ring atoms, aromatic groups having 6 to 20 ring atoms, heteroaryl groups having 5 to 20 ring atoms, -NR'R", silyl group, carbonyl group, alkoxycarbonyl group, aryloxycarbonyl group, carbamoyl group, haloformyl group, formyl group, isocyanate group, thiocyanate group, isothiocyanate group, hydroxyl group, trifluoromethyl group, and the above groups may be further substituted by substituents acceptable in the art. It can be understood that R' and R" in -NR'R" are each independently selected from, but not limited to, H, deuterium atom, cyano group, isocyano group, nitro group or halogen, alkyl groups having 1 to 10 carbon atoms, heterocyclyl groups having 3 to 20 ring atoms, aromatic groups having 6 to 20 ring atoms, heteroaryl groups having 5 to 20 ring atoms. Preferably, R is selected from, but not limited to, deuterium atom, cyano group, isocyano group, nitro group or halogen, alkyl groups having 1 to 10 carbon atoms, heterocyclyl groups having 3 to 10 ring atoms, aromatic groups having 6 to 20 ring atoms, heteroaryl groups having 5 to 20 ring atoms, silyl group, carbonyl group, alkoxycarbonyl group, aryloxycarbonyl group, carbamoyl group, haloformyl group, formyl group, isocyanate group, thiocyanate group, isothiocyanate group, hydroxyl group, trifluoromethyl group, and the above groups may be further substituted by substituents acceptable in the art.

In the present application, "the number of ring atoms" refers to the number of atoms constituting the ring itself in a structural compound (e.g., a monocyclic compound, a fused ring compound, a cross-linked compound, a carbocyclic compound, a heterocyclic compound) in which atoms are bonded to form a ring. In a case that the ring is replaced by a substituent, the atoms contained in the substituent are not included in the ring-forming atoms. The "number of ring atoms" mentioned below has the same meaning unless otherwise specified, for example, the number of ring atoms of benzene ring is 6, the number of ring atoms of naphthalene ring is 10, and the number of ring atoms of thienyl group is 5.

In the present application, "aryl groupsor aromatic group" refers to an aromatic hydrocarbon group derived from an aromatic ring compound by removing one hydrogen atom. It may be a monocyclic aryl group, a fused ring aryl group or a polycyclic aryl group, and for polycyclic rings, at least one of them is an aromatic ring system. For example, "substituted or unsubstituted aryl groups having 6 to 40 ring atoms" refers to aryl groups having 6 to 40 ring atoms, preferably substituted or unsubstituted aryl groups having 6 to 30 ring atoms, more preferably substituted or unsubstituted aryl groups having 6 to 18 ring atoms, particularly preferably substituted or unsubstituted aryl groups having 6 to 14 ring atoms, and optionally the above aryl groups may be further substituted. Suitable examples include, but are not limited to, phenyl, biphenyl, terphenyl, naphthyl, anthracenyl, phenanthrenyl, fluoranthenyl, triphenylene, pyrenyl, perylene, tetraphenyl, fluorenyl, perylene, acenaphthylene, and derivatives thereof. It can be understood that a plurality of aryl groups may also be interrupted by short non-aromatic units (for example, < 10% of non-H atoms, such as C, N or O atoms), such as acenaphthylene, fluorene, or 9,9-diarylfluorene, triarylamine, diaryl ether systems should also be included in the definition of aryl groups.

In the present application, "heteroaryl group or heteroaromatic group" means that at least one carbon atom is replaced by a non-carbon atom on the basis of an aryl group. The non-carbon atom can be an N atom, an O atom, an S atom, or the like. For example, "substituted or unsubstituted heteroaryl groups having 5 to 40 ring atoms" refers to heteroaryl groups having 5 to 40 ring atoms, preferably substituted or unsubstituted heteroaryl groups having 6 to 30 ring atoms, more preferably substituted or unsubstituted heteroaryl groups having 6 to 18 ring atoms, particularly preferably substituted or unsubstituted heteroaryl groups having 6 to 14 ring atoms, and optionally the heteroaryl groups can be further substituted. Ssuitable examples include, but are not limited to: thienyl, furanyl, pyrrolyl, imidazolyl, diazolyl, triazolyl, imidazolyl, pyridyl, bipyridyl, pyrimidinyl, triazinyl, acridinyl, pyridazinyl, pyrazinyl, quinolinyl, isoquinolinyl, quinazolinyl, quinoxalinyl, phthalazinyl, pyridopyrimidinyl, pyridopyrazinyl, benzothienyl, benzofuranyl, indolyl, pyrroloimidazolyl, pyrrolopyrrolyl, thienopyrrolyl, thienothienyl, furopyrrolyl, furofuranyl, thienofuranyl, benzisoxazolyl, benzisothiazolyl, benzimidazolyl, o-naphthyridinyl, phenanthrenyl, primary pyridyl, quinazolinonyl, dibenzothienyl, dibenzofuranyl, carbazolyl and derivatives thereof.

In the present application, "alkyl group" may represent a linear, branched and/or cyclic alkyl group. The number of carbons in an alkyl group can be 1 to 50, 1 to 30, 1 to 20, 1 to 10, or 1 to 6. A phrase containing the term, for example, "C₁₋₉ alkyl group" refers to an alkyl group containing 1 to 9 carbon atoms, which may independently be a C₁ alkyl group, a C₂ alkyl group, a C₃ alkyl group, a C₄ alkyl group, a C₅ alkyl group, a C₆ alkyl group, a C₇ alkyl group, a C₈ alkyl group, or a C₉ alkyl group. Non-limiting examples of alkyl groups include methyl, ethyl, n-propyl, isopropyl, n-butyl, sec-butyl, tert-butyl, isobutyl, 2-ethylbutyl, 3,3-dimethylbutyl, n-pentyl, isopentyl, neopentyl, tert-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butylhexyl, cyclohexyl, 4-methylcyclohexyl, 4-tert-butylcyclohexyl, n-heptyl, 1-methylheptyl, 2,2-dimethylheptyl, 2-ethylheptyl, 2-butylheptyl, n-octyl, tert-octyl, 2-ethyloctyl, 2-butyloctyl, 2-hexyloctyl, 3,7-dimethyloctyl, cyclooctyl, n-nonyl, n-decyl, adamantyl, 2-ethyldecyl, 2-butyldecyl, 2-hexyldecyl, 2-octyldecyl, n-undecyl, n-dodecyl, 2-ethyldodecyl, 2-butyldodecyl, 2-hexyldodecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyleicosyl, 2-octyleicosyl, n-heneicosyl, n-docosyl, n-tricosyl, n-tetracosyl, n-pentacosyl, n-hexacosyl, n-heptacosyl, n-octacosyl, n-hexacosyl, n-triacontane, amantadane, etc.

In the present application, the abbreviations of the substituents shall correspond to n-normal; sec-secondary, i-iso, t-tertiary, o-ortho, m-methyl, p-phenol, Me-methyl, Et-etethyl, Pr-propyl, Bu-butyl, Am-n-pentyl, Hx-hexyl, Cy-cyclohexyl.

In the present application, "amino" refers to a derivative of an amine having the structural characteristics of formula -N(X)₂, wherein each "X" is independently selected from H, substituted or unsubstituted alkyl, substituted or unsubstituted cycloalkyl, substituted or unsubstituted heterocyclyl, and the like. Non-limiting types of amine groups include -NH₂, -N(alkyl)₂, -NH(alkyl), -N(cycloalkyl)₂, -NH(cycloalkyl), -N(heterocyclyl)₂, -NH(heterocyclyl), -N(aryl)₂, -NH(aryl), -N(alkyl)(aryl), - N(alkyl)(heterocyclyl), -N(cycloalkyl)(heterocyclyl), -N(aryl)(heteroaryl), -N(alkyl)(heteroaryl),or the like.

In the present application, unless otherwise defined, hydroxyl refers to -OH, carboxyl refers to -COOH, carbonyl refers to-C(=O)-, amino refers to -NH₂, formyl refers to -C(=O)H, haloformyl refers to -C(=O)Z (where Z represents halogen), carbamoyl refers to -C(=O)NH₂, isocyanate refers to -NCO, and isothiocyanate refers to -NCS.

In the present application, the term "alkoxy" refers to a group having the structure "-O-alkyl", i.e., an alkyl group as defined above is attached to other groups via an oxygen atom. Suitable examples of phrases including the term include, but are not limited to, methoxy (-O-CH₃ or -OMe), ethoxy (-O-CH₂CH₃ or -OEt), and tert-butoxy (-O-C(CH₃)₃ or -OtBu).

In the present application, "*" indicates a linkage site or a fusion site.

In the present application, in a case that the linkage site is not specified in a group, it means that any linkage site in a group can be used as a linkage site.

In the present application, in a case that the fusion site is not specified in a group, it means that any fusion site in a group can be used as a fusion site, preferably two or more sites in the adjacent position of the group are fusion sites.

In the present application, the fused site acts as a fused site, preferably two or more sites in the ortho position of the group are fused sites;

In the present application, when the same group contains multiple substituents with identical symbol, each substituent may be the same or different from each other, for example, for 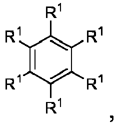 six R¹ on the phenyl ring may be the same or different from each other.

In the present application, a single bond connected to a substituent extends through the corresponding ring, meaning that the substituent may be connected to any position of the ring, for example, R in 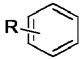 may be connected to any substitutable position of the phenyl ring. For example, 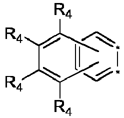 denotes that 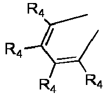 may be formed a ring with an optional substitutable position on

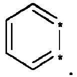

The technical solutions of the present application are as follows:

An arylamine organic compound having a structure represented by formula (1): 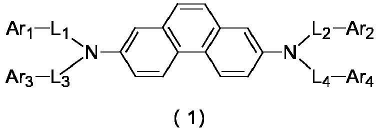 wherein:
L₁ to L₄ are each independently selected from a single bond, a substituted or unsubstituted aromatic group having 6 to 30 ring atoms, or a substituted or unsubstituted heteroaromatic group having 5 to 30 ring atoms;
An is selected from any structure represented by formula (A-1) or formula (A-2);
Ar₂ is selected from any structure represented by formulas (A-2) to (A-6);
Ar₃ and Ar₄ are each independently selected from any structure represented by formulas (A-3) to (A-6) described above;
structures represented by formulas (A-1) to (A-6) are as follows: 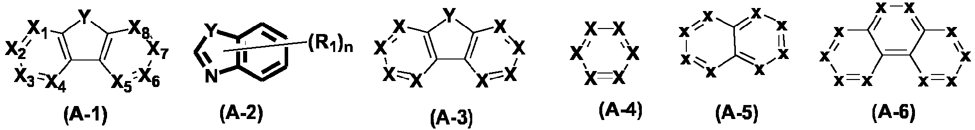
n is selected from 0, 1, 2, 3 or 4;
Y is selected from O, S or NR₂;
X and X₁ to X₈ are each independently selected from CR₃ or N, respectively, and at least one of X₁ to X₈ is selected from N; and;
R₁, R₂ and R₃ are each independently selected from -H, -D, a linear alkyl group having 1 to 20 carbon atoms, a linear alkoxy group having 1 to 20 carbon atoms, a branched alkyl group having 3 to 20 carbon atoms, a branched alkoxy group having 3 to 20 carbon atoms, a cyclic alkyl group having 3 to 20 carbon atoms, a cyclic alkoxy group having 3 to 20 carbon atoms, a substituted or unsubstituted silyl group, a cyano group, an isocyano group, a hydroxyl group, a nitro group, an amino group, -CF₃, -Cl, -Br, -F, -I, an alkenyl group having 2 to 20 carbon atoms, or a substituted or unsubstituted aromatic group having 6 to 20 ring atoms, or a combination thereof.

In an embodiment, R₁, R₂ and R₃ are each independently selected from -H, -D, a linear alkyl group having 1 to 10 carbon atoms, a linear alkoxy group having 1 to 10 carbon atoms, a branched alkyl group having 3 to 10 carbon atoms, a branched alkoxy group having 3 to 10 carbon atoms, a cyclic alkyl group having 3 to 10 carbon atoms, a cyclic alkoxy group having 3 to 10 carbon atoms, a substituted or unsubstituted silyl group, a cyano group, an isocyano group, a hydroxyl group, a nitro group, an amine group, -CF₃, -Cl, -Br, -F, -I, an alkenyl group having 2 to 10 carbon atoms, or a substituted or unsubstituted aromatic group having 6 to 10 ring atoms, or a combination of thereof.

In an embodiment, the formula (1) is selected from any one of the structures represented by formulas (2- 1) to (2- 6): 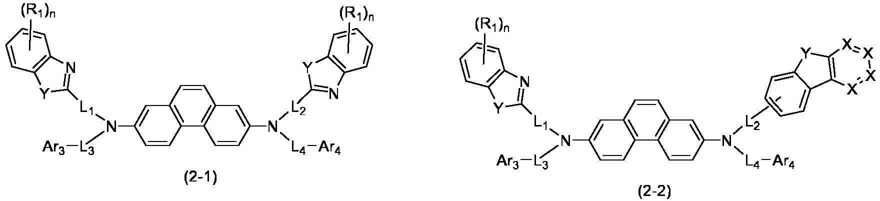 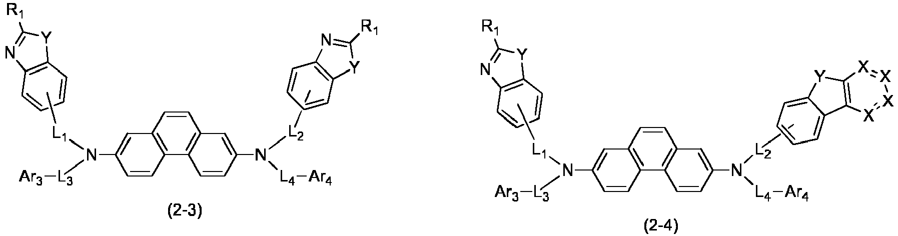 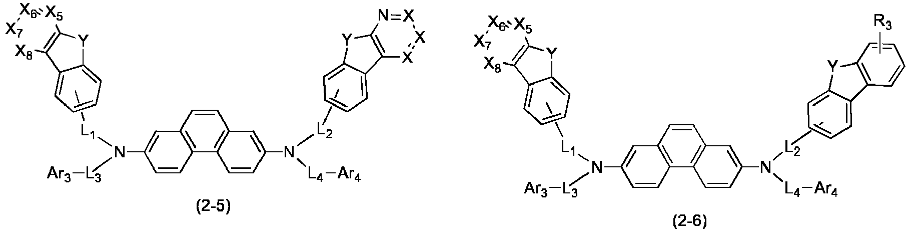 wherein R₁, R₃, Y, X, X₅ to X₈, L₁ to L₄, Ar₃, and Ar₄ have the same meanings as described above.

In an embodiment, in formulas (2-5) and (2-6), X₅ to X₈ are each independently selected from CR₃ or N, and at least one of X₅ to X₈ is selected from N. Preferably, X₅ is selected from N.

In an embodiment, in a case that Y appears multiple times in formulas (2-1), (2-3), and (2-5), it is selected from the same group.

In an embodiment, X appears multiple times in formulas (2-2), (2-4), and (2-6), it is independently selected from CR₃. Furthermore, R₃ is independently selected from -H, -D, a linear alkyl group having 1 to 10 carbon atoms, a branched alkyl group having 3 to 10 carbon atoms, a cyclic alkyl group having 3 to 10 carbon atoms, a substituted or unsubstituted silyl group, a cyano group, an isocyano group, a hydroxy group, a nitro group, a -CF₃, -Cl, -Br, -F, -I, or a substituted or unsubstituted aromatic group having 6 to 10 ring atoms, or a combination of thereof.

In an embodiment, in formulas (2-1) to (2-4), R₁ is independently selected from -H, -D, a linear alkyl group having 1 to 10 carbon atoms, a branched alkyl group having 3 to 10 carbon atoms, a cyclic alkyl group having 3 to 10 carbon atoms, or a substituted or unsubstituted aromatic group having 6 to 10 ring atoms, or a combination of thereof.

In an embodiment, L₁ to L₄ are each independently selected from a single bond, a substituted or unsubstituted aromatic group having 6 to 20 ring atoms, or a substituted or unsubstituted heteroaromatic group having 6 to 20 ring atoms.

Furthermore, L₁ to L₄ may be selected from a single bond, a substituted or unsubstituted aromatic group having 6 to 13 ring atoms, or a substituted or unsubstituted heteroaromatic group having 6 to 13 ring atoms.

In an embodiment, L₁ to L₄ are each independently selected from a single bond, a substituted or unsubstituted phenyl, a substituted or unsubstituted biphenyl, a substituted or unsubstituted terphenyl, a substituted or unsubstituted naphthyl, a substituted or unsubstituted anthryl, a substituted or unsubstituted phenanthrenyl, a substituted or unsubstituted fluoranthenyl, a substituted or unsubstituted triphenylene, a substituted or unsubstituted pyrenyl, a substituted or unsubstituted perylene, a substituted or unsubstituted tetraphenyl, a substituted or unsubstituted fluorenyl, a substituted or unsubstituted perylene, a substituted or unsubstituted acenaphthylene, a substituted or unsubstituted thienyl, a substituted or unsubstituted furyl, a substituted or unsubstituted pyrrolyl, a substituted or unsubstituted imidazolyl, a substituted or unsubstituted triazolyl, a substituted or unsubstituted diazolyl, a substituted or unsubstituted pyridyl, a substituted or unsubstituted bipyridyl, a substituted or unsubstituted pyrimidinyl, a substituted or unsubstituted triazinyl, a substituted or unsubstituted acridinyl, a substituted or unsubstituted pyridazinyl, a substituted or unsubstituted pyrazinyl, a substituted or unsubstituted quinolinyl, a substituted or unsubstituted quinazolinyl, a substituted or unsubstituted quinoxalinyl, a substituted or unsubstituted phthalazinyl, a substituted or unsubstituted isoquinolinyl, a substituted or unsubstituted indolyl, a substituted or unsubstituted benzothienyl, a substituted or unsubstituted benzofuranyl, or a substituted or unsubstituted 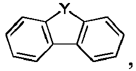 in which Y has the same meaning as described above.

The "substituted or unsubstituted" has the same meaning as described above.

In an embodiment, "substituted" in "substituted or unsubstituted" refers to a substituent that is selected from a deuterium atom, a cyano group, an isocyano group, a nitro group, or a halogen, an alkyl group having 1 to 8 carbon atoms, a heterocyclic group having 3 to 8 ring atoms, an aromatic group having 6 to 10 ring atoms, or a heteroaromatic group having 6 to 10 ring atoms.

Further, L₁ to L₄ are each independently selected from a single bond or any one of the following groups: 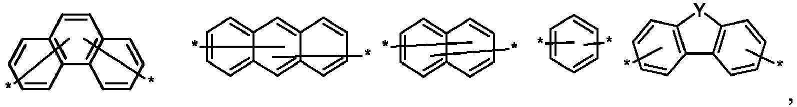 wherein * indicates a linkage site.

In an embodiment, Y is selected from O or S.

In an embodiment, L₁ to L₄ are each independently selected from a single bond or a phenyl group, respectively, and further, the formula (1) is selected from any structure represented by formulas (3-1) to (3-6): 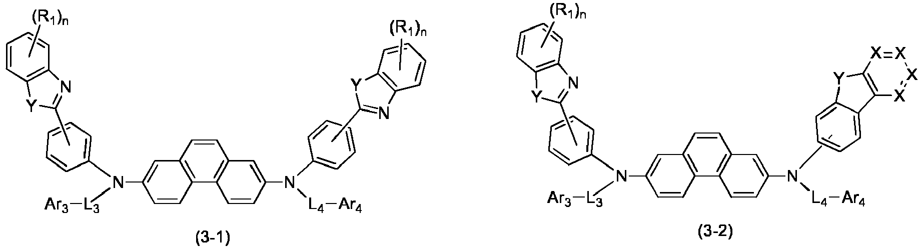 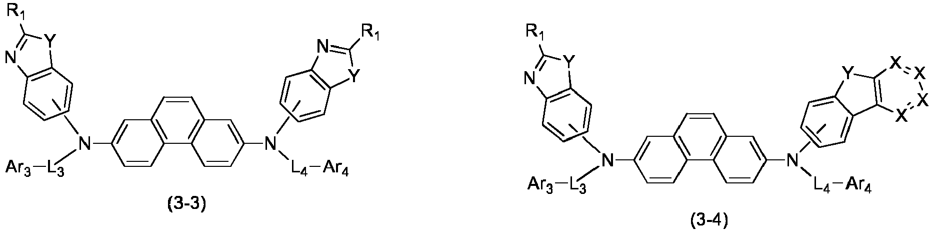 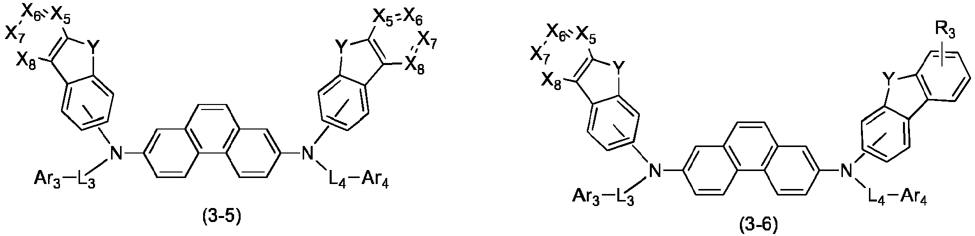 wherein R₁, Y, X, X₅ to X₈, L₃ to L₄, Ar₃ and Ar₄ have the same meanings as described above.

In an embodiment, Y in the formulas (3-1) to (3-4) is selected from O or S. In some embodiments, Y is selected from oxygen.

In an embodiment, X₅ in formulas (3-5) and (3-6) is selected from N.

In an embodiment, formula (1) is selected from any one of the formulas (4-1) to (4-6):

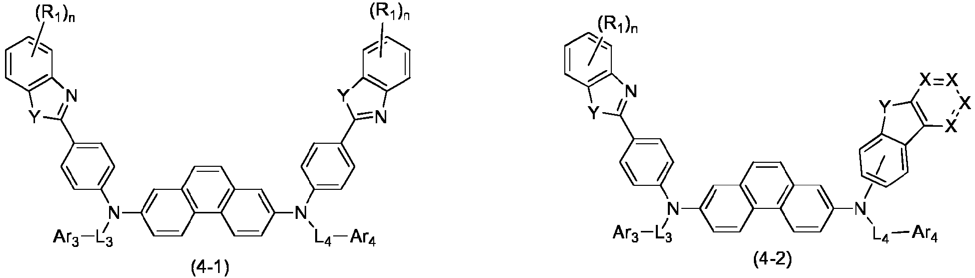

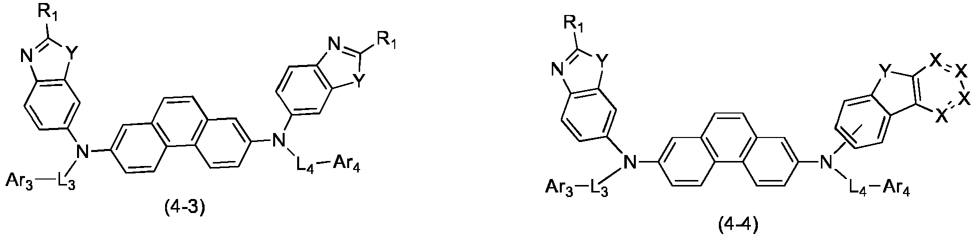

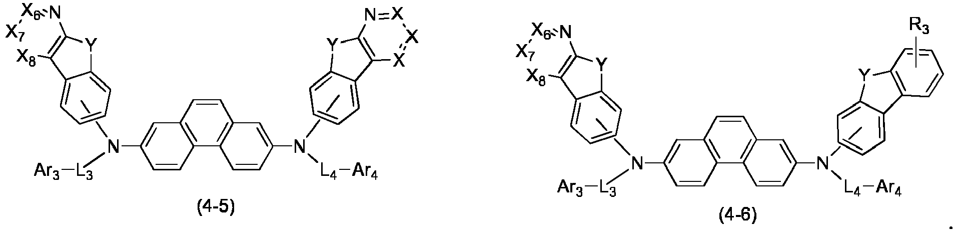

In an embodiment, in a case that Ar₁ and Ar₂ are selected from the same group, the organic compounds described in the present application are selected from symmetric structures.

In an embodiment, Ar₃ and Ar₄ are each independently selected from any one of the groups represented by formulas (B-1) to (B-7): 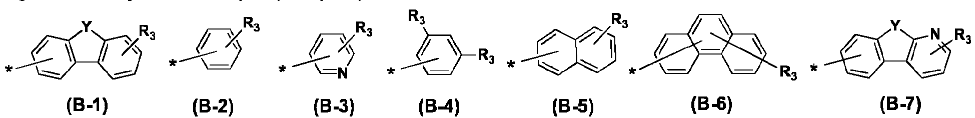 wherein * indicates a linkage site; and
R₃ is independently selected from -H, -D, a linear alkyl group having 1 to 10 carbon atoms, a branched alkyl group having 3 to 10 carbon atoms, a cyclic alkyl group having 3 to 10 carbon atoms, a silyl group, a cyano group, an isocyano group, a hydroxyl group, a nitro group, -CF₃, -Cl, -Br, -F, -I, phenyl, biphenyl, terphenyl, or naphthyl.

In an embodiment, *-L₃-Ar₃ and/or *-L₄-Ar₄ are selected from the following groups:

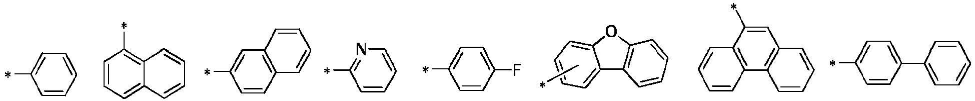

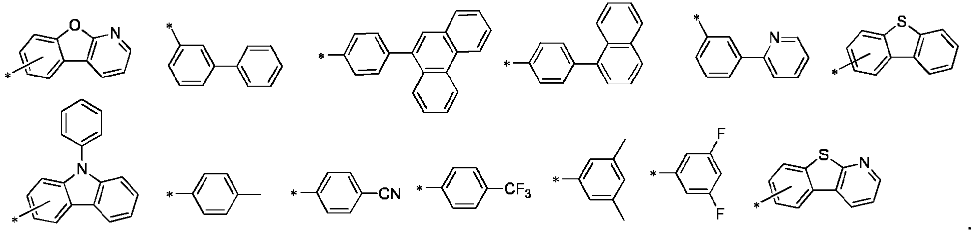

In an embodiment, *-L₃-Ar₃ and/or *-L₄-Ar₄ are selected from the same group.

In an embodiment, at a wavelength 630 nm, the arylamine organic compound according to the present application has a refractive index greater than 1.7; preferably greater than 1.78; and more preferably greater than 1.83.

In an embodiment, the arylamine organic compounds according to the present application have a small extinction coefficient at a wavelength greater than 430 nm and a high transmittance for visible light, thereby reducing the effect on the luminous efficiency of the device. At a wavelength of 430 nm, the arylamine organic compound has an extinction coefficient of less than 0.1; preferably, less than 0.003; and more preferably less than 0.001.

In some embodiments, the arylamine organic compounds according to the present application have a large extinction coefficient in the wavelength range of less than or equal to 400 nm. As an example, at a wavelength of 350 nm, the extinction coefficient of the arylamine organic compounds is greater than or equal to 0.3; preferably greater than or equal to 0.5, more preferably greater than or equal to 0.7, and most preferably greater than or equal to 1.0.

In some embodiments, the arylamine organic compounds according to the present application have a higher glass transition temperature to enhance the thermal stability of the materials. In some embodiments, the arylamine organic compound has a glass transition temperature Tg, which is greater than or equal to 100°C; preferably Tg is greater than or equal to 120°C, more preferably Tg is greater than or equal to 140°C, and more preferably Tg is greater than or equal to 160°C.

As an example, the arylamine organic compounds according to the present application may be selected from but not limited to any one of the following structures:

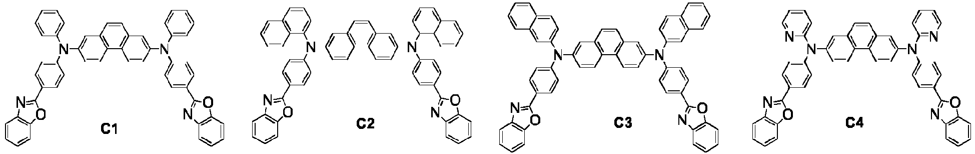

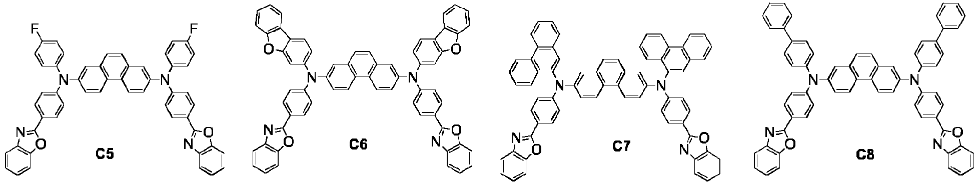

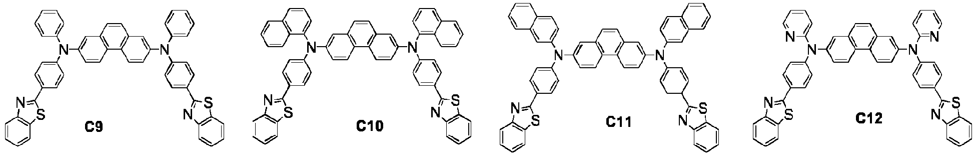

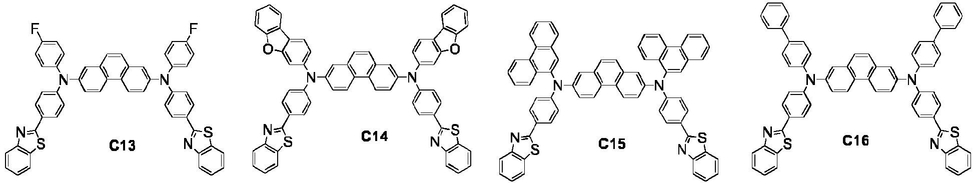

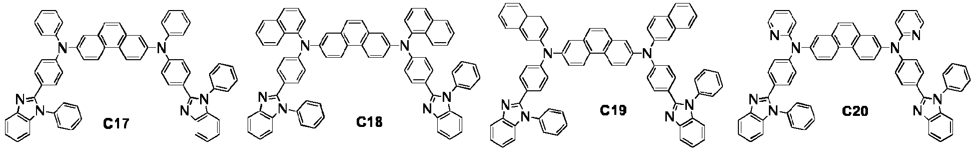

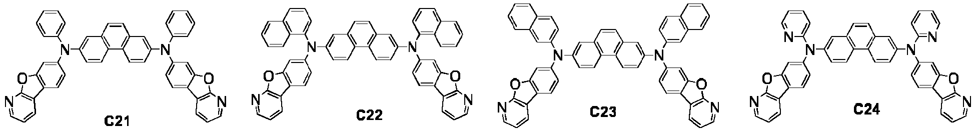

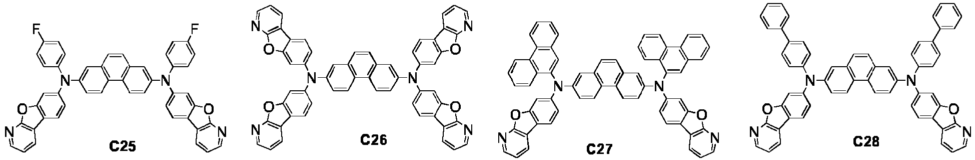

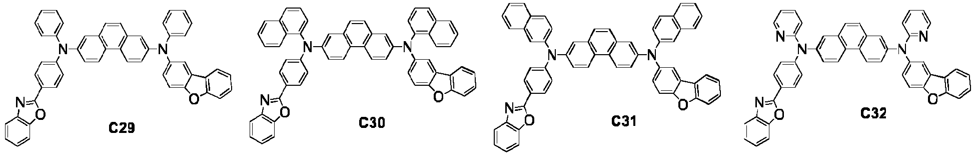

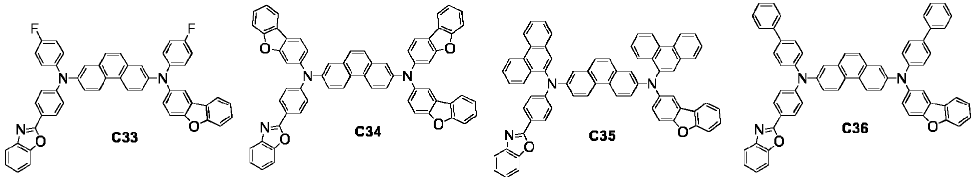

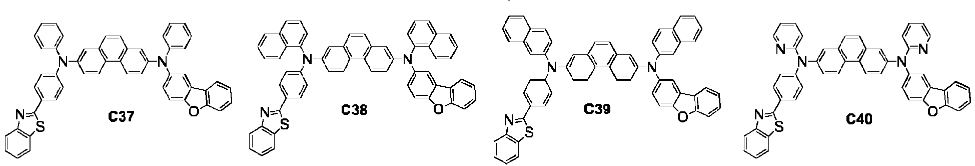

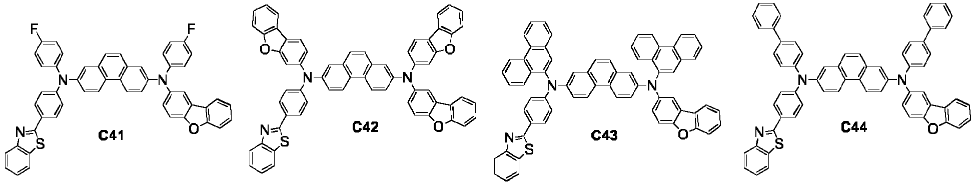

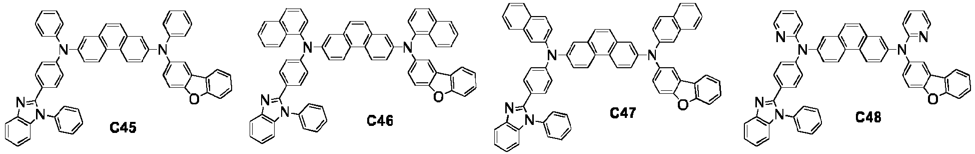

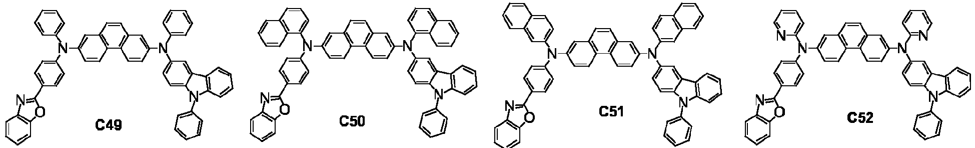

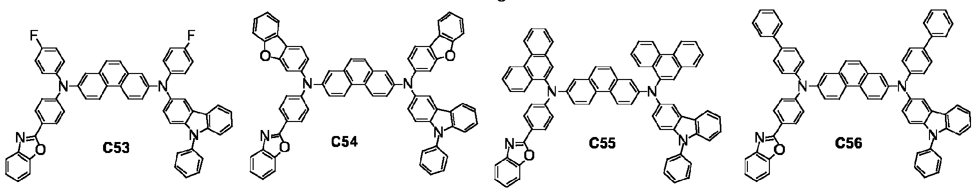

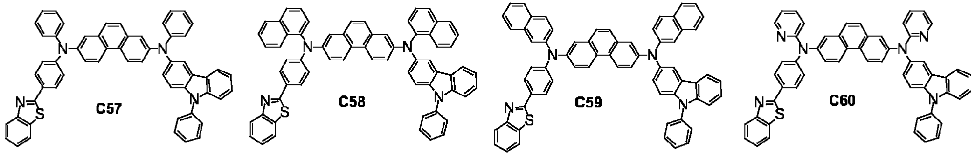

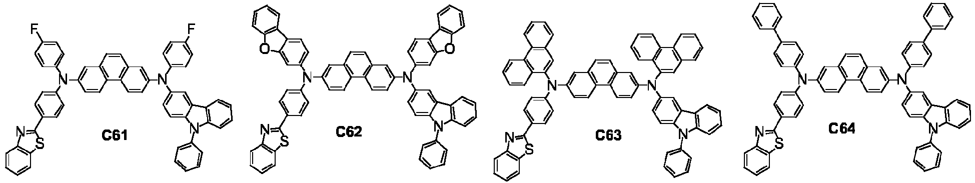

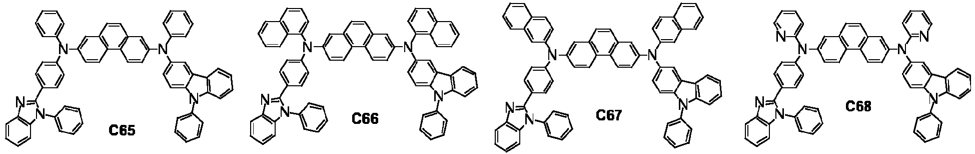

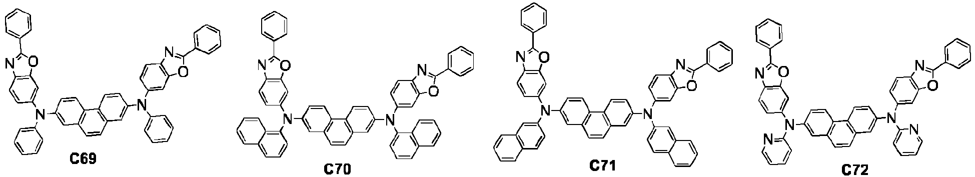

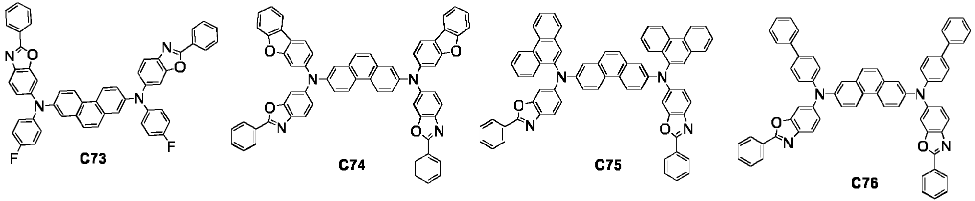

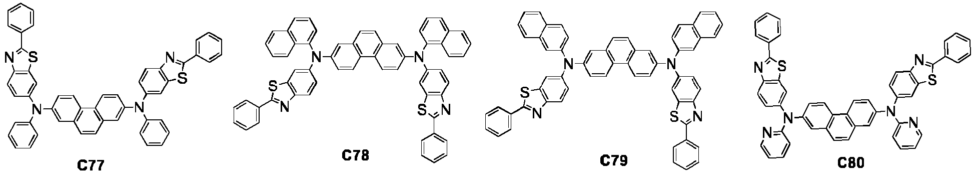

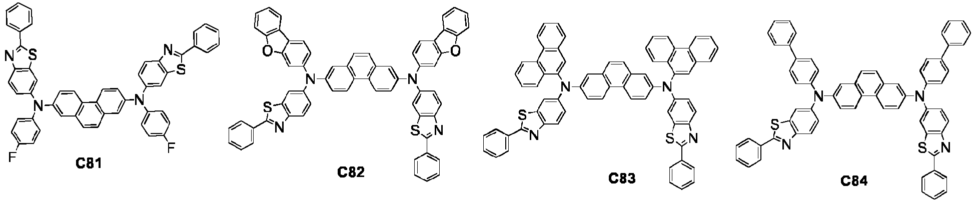

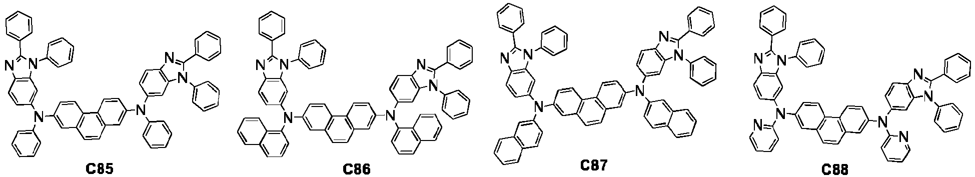

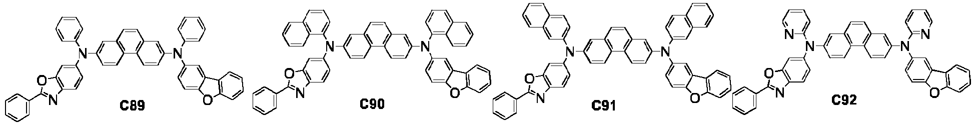

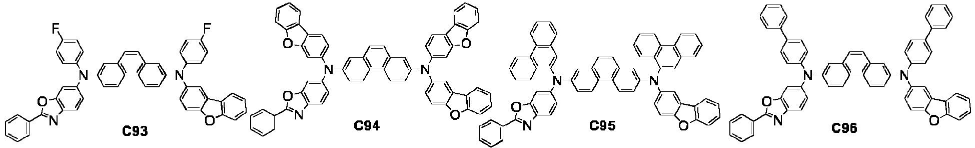

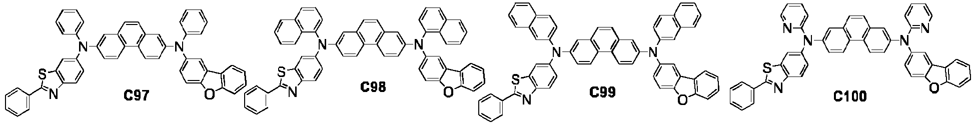

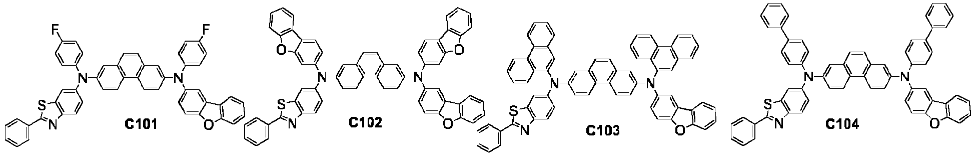

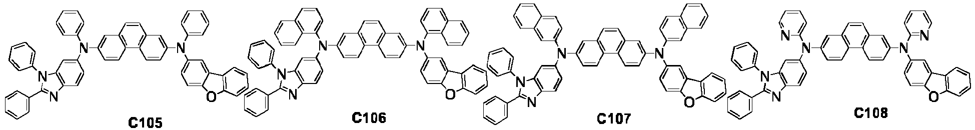

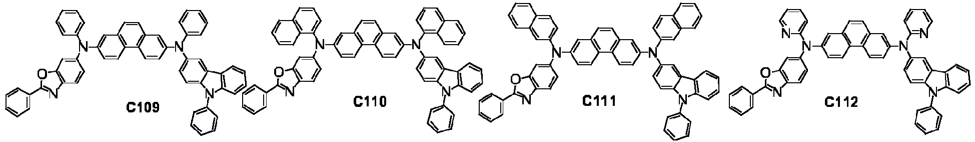

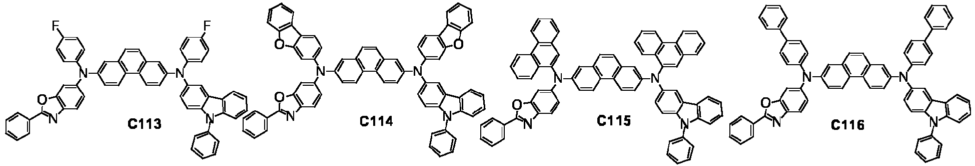

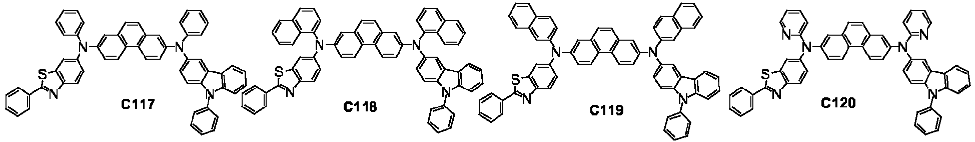

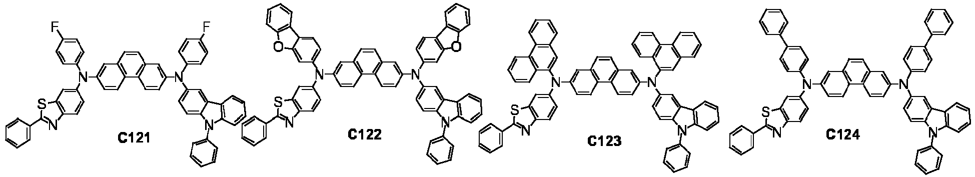

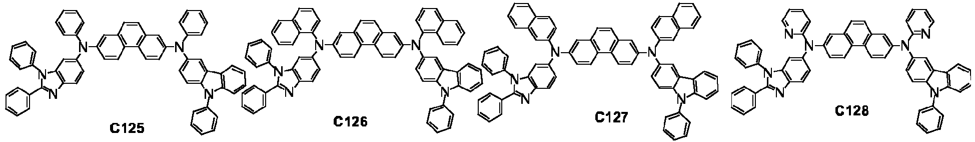

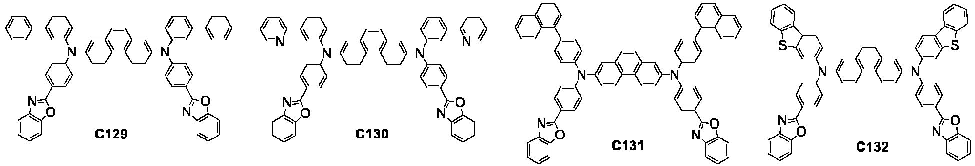

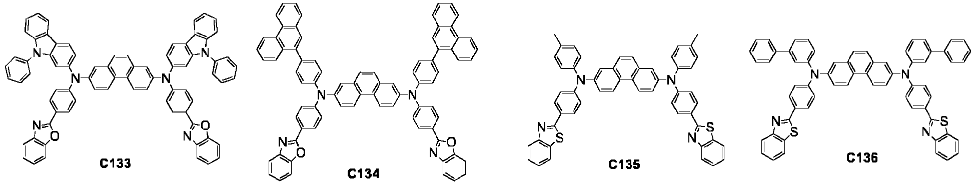

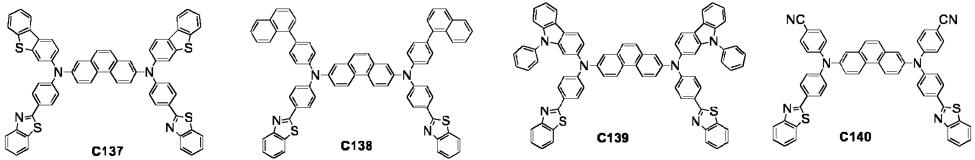

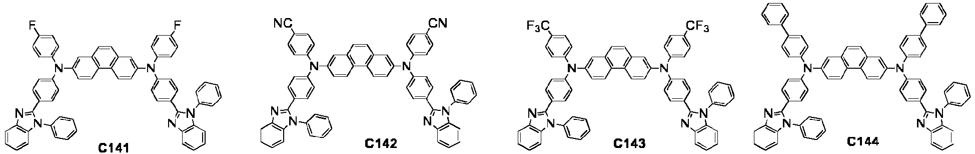

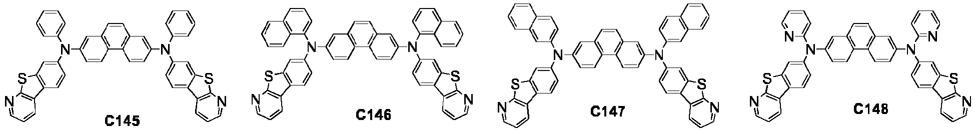

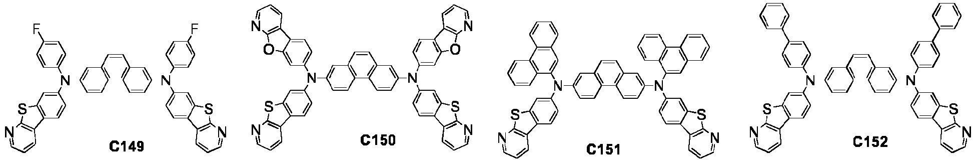

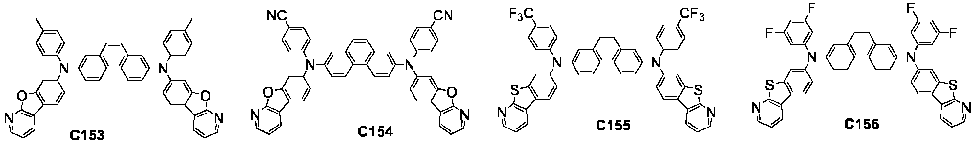

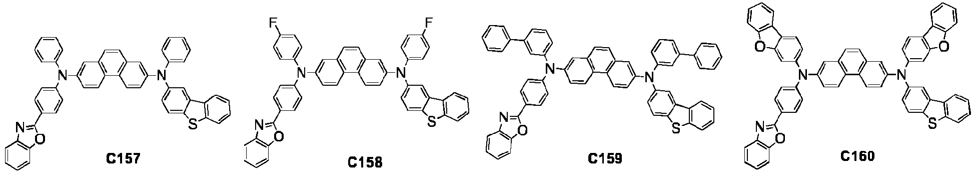

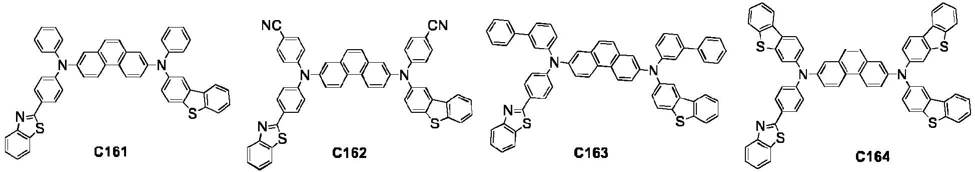

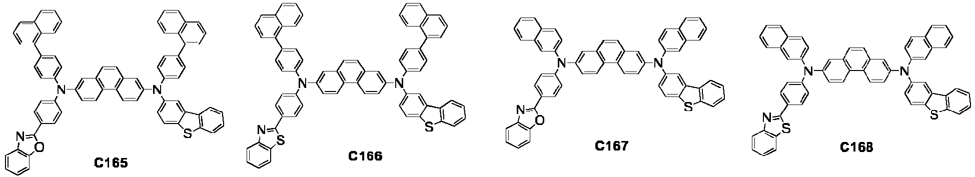

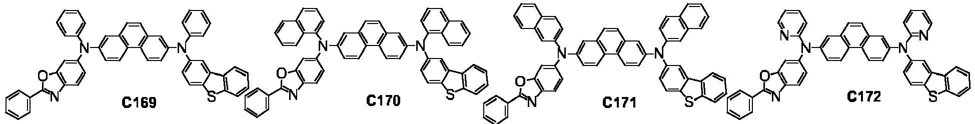

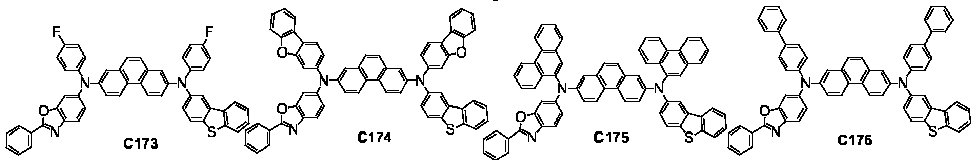

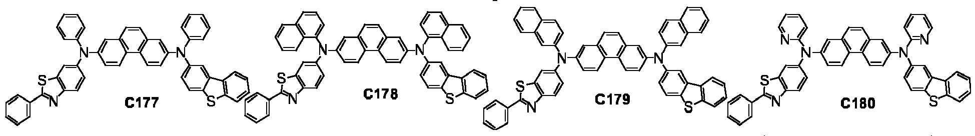

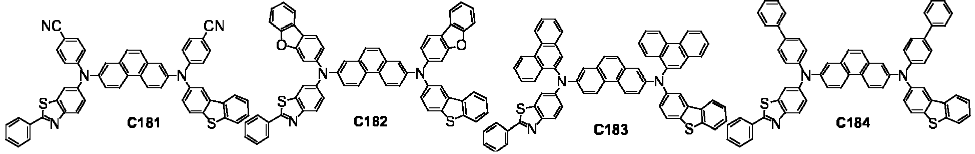

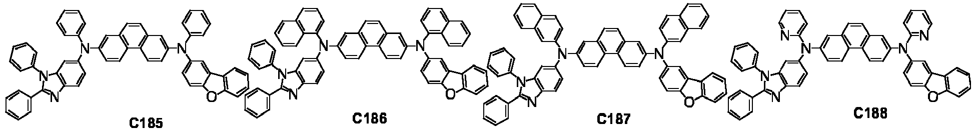

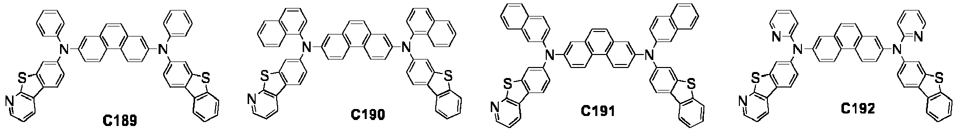

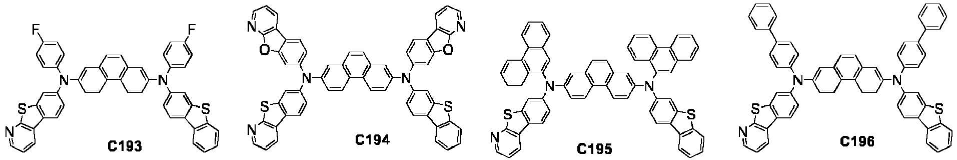

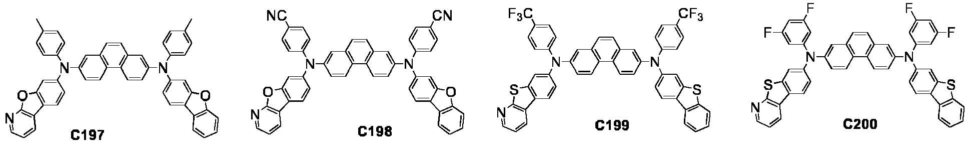

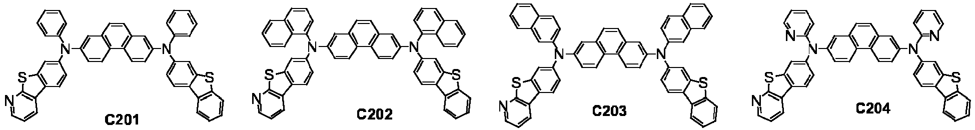

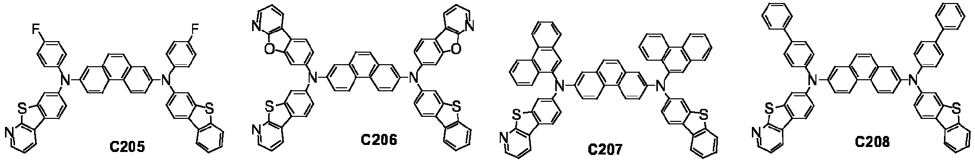

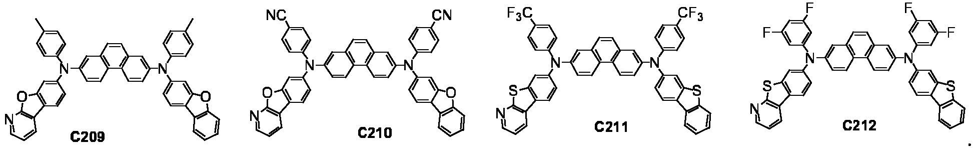

It can be understood that H in the structural formula of the arylamine organic compounds described above may be further substituted.

The present application further relates to a mixture including at least one of the above-mentioned organic compounds and at least another organic functional material. The at least another organic functional material may be, but is not limited to, a hole injection material (HIM), a hole transport material (HTM), an electron transport material (ETM), an electron injection material (EIM), an electron blocking material (EBM), a hole blocking material (HBM), a light-emitting material (EIM), a host material (Host), or an organic dye. Various organic functional materials are described in detail, for example, in WO2010135519A1, US20090134784A1, and WO2011110277A1, and the entire contents of which are hereby incorporated herein by reference.

The present application also relates to a composition including at least one arylamine organic compound or mixture as described above, and at least one organic solvent.

The organic solvent is selected from at least one of an aromatic or heteroaromatic-based solvent, an ester-based solvent, an aromatic ketone-based solvent, an aromatic ether-based solvent, an aliphatic ketone, an aliphatic ether, an alicyclic compound, an olefin-based compound, a borate-based compound, and a phosphate-based compound.

In at least one embodiment, in the composition, the organic solvent is selected from an aromatic or heteroaromatic-based solvent.

The aromatic or heteroaromatic-based solvents suitable for the present application are, but are not limited to, at least one of p-diisopropylbenzene, pentylbenzene, tetrahydronaphthalene, cyclohexylbenzene, chloronaphthalene, 1, 4-dimethylnaphthalene, 3-isopropylbiphenyl, p-methylcumene, dipentylbenzene, tripentylbenzene, amyl-toluene, o-diethylbenzene, m-diethylbenzene, p-diethylbenzene, 1,2,3,4-tetramethylbenzene, 1,2,3,5-tetramethylbenzene, 1,2,4,5-tetramethylbenzene, butylbenzene, dodecylbenzene, dihexylbenzene, dibutylbenzene, p-diisopropylbenzene, cyclohexylbenzene, benzylbutylbenzene, dimethylnaphthalene, 3-isopropylbiphenyl, p-methylcumene, 1-methylnaphthalene, 1,2,4-trichlorobenzene, 4,4-difluorodiphenylmethane, 1,2-dimethoxy-4-(1-propenyl)benzene, diphenylmethane, 2-phenylpyridine, 3-phenylpyridine, N-methyldiphenylamine, 4-isopropylbiphenyl, α,α-dichlorodiphenylmethane, 4-(3-phenylpropyl)pyridine, benzyl benzoate, 1,1-bis(3,4-dimethylphenyl)ethane, 2-isopropylnaphthalene, quinoline, isoquinoline, methyl 2-furanoate, ethyl 2-furanoate, and the like.

The ester-based solvents may be selected from, but not limited to, alkyl octanoate, alkyl sebacate, alkyl stearate, alkyl benzoate, alkyl phenylacetate, alkyl cinnamate, alkyl oxalate, alkyl maleate, alkyl lactone, alkyl oleate, and the like. At least one of octyl octanoate, diethyl sebacate, diallyl phthalate, and isononyl isononanoate is particularly preferred.

The aromatic ketone-based solvents may be selected from, but not limited to, 1-tetralone, 2-tetralone, 2-(phenylepoxy) tetralone, 6-(methoxy) tetralone, acetophenone, propiophenone, benzophenone, and derivatives thereof. As an example, the derivative may be selected from at least one of 4-methylacetophenone, 3-methylacetophenone, 2-methylacetophenone, 4-methylacetophenone, 3-methylacetophenone and 2-methylacetophenone.

The aromatic ether-based solvents may be selected from, but is not limited to, at least one of 3-phenoxytoluene, butoxybenzene, p-anisaldehyde dimethyl acetal, tetrahydro-2-phenoxy-2H-pyran, 1,2-dimethoxy-4-(1-propenyl)benzene, 1,4-benzodioxane, 1,3-dipropylbenzene, 2,5-dimethoxytoluene, 4-ethylbenzoethyl ether, 1,3-dipropoxybenzene, 1,2,4-trimethoxybenzene, 4-(1-propenyl)-1,2-dimethoxybenzene, 1,3-dimethoxybenzene, glycidyl phenyl ether, dibenzyl ether, 4-tert-butyl anisole, trans-p-propenyl anisole, 1,2-dimethoxybenzene, 1-methoxynaphthalene, diphenyl ether, 2-phenoxymethyl ether, 2-phenoxytetrahydrofuran and ethyl-2-naphthyl ether.

The aliphatic ketone-based solvents may be selected from, but not limited to, at least one of 2-nonanone, 3-nonanone, 5-nonanone, 2-decanone, 2,5-hexanedione, 2,6,8-trimethyl-4-nonanone, fenchone, phorone, isophorone, di-n-amyl ketone, and the like; or aliphatic ethers, such as amyl ether, hexyl ether, dioctyl ether, ethylene glycol dibutyl ether, diethylene glycol diethyl ether, diethylene glycol butyl methyl ether, diethylene glycol dibutyl ether, triethylene glycol dimethyl ether, triethylene glycol ethyl methyl ether, triethylene glycol butyl methyl ether, tripropylene glycol dimethyl ether and tetraethylene glycol dimethyl ether.

It can be understood that the organic solvent may be used alone or as a mixed solvent of two or more organic solvents.

In an embodiment, the composition of the present application includes at least one arylamine organic compound or mixture as described above, and at least one organic solvent, and may further includes another organic solvent.

Said another organic solvent may be selected from, but is not limited to, at least one of methanol, ethanol, 2-methoxyethanol, dichloromethane, trichloromethane, chlorobenzene, o-dichlorobenzene, tetrahydrofuran, anisole, morpholine, toluene, o-xylene, m-xylene, p-xylene, 1, 4-dioxane, acetone, methyl ethyl ketone, 1,2-dichloroethane, 3-phenoxytoluene, 1,1,1 -trichloroethane, 1,1,2,2-tetrachloroethane, ethyl acetate, butyl acetate, dimethylformamide, dimethylacetamide, dimethylsulfoxide, tetrahydronaphthalene, decalin, and indene.

In some preferred embodiments, an organic solvent suitable for the present application is a solvent having a Hansen solubility parameter in the following ranges:
δd (dispersion force): ranges from 17.0 MPa^{1/2} to 23.2 MPa^{1/2}, especially ranges from 18.5 MPa^{1/2} to 21.0 MPa^{1/2};
Δp (polarity force) ranges from 0.2 MPa^{1/2} to 12.5 MPa^{1/2}, especially ranges from 2.0 MPa^{1/2} to 6.0 MPa^{1/2};
Δh (hydrogen bonding force) ranges from 0.9 MPa^{1/2} to 14.2 MPa^{1/2}, especially ranges from 2.0 MPa^{1/2} to 6.0 MPa^{1/2}.

In an embodiment, for the compositions in the present application, the boiling point should be considered when selecting an organic solvent. In at least some embodiments, the boiling point of the organic solvent is greater than or equal to 150 °C; preferably greater than or equal to 180 °C; preferably, greater than or equal to 200 °C; more preferably, greater than or equal to 250 °C; and most preferably greater than or equal to 300 °C. Boiling points in these ranges are beneficial to prevent nozzle clogging of inkjet print heads.

It can be understood that the organic solvent can be evaporated from that solvent system to form a thin film containing the organic compounds.

In an embodiment, the composition is a solution. In still other embodiments, the composition is a suspension. The solution or suspension may additionally include additives for adjusting viscosity, film-forming performance, improving adhesion, and the like. The additives may be selected from, but not limited to, at least one of a surface active compound, a lubricant, a wetting agent, a dispersing agent, a hydrophobic agent, and an adhesive.

The content of the arylamine organic compound in the composition ranges from 0.01wt% to 10wt%, preferably from 0. 1wt% to 8wt%, more preferably from 0.2wt% to 5wt%, and most preferably from 0.25wt% to 3wt%.

The present application also relates to the use of the compositions as coatings or printing inks in the manufacture of organic electronic devices. In an embodiment, the composition is used to manufacture an organic electronic device through printing or coating. The preparation method of the printing or coating may be, but is not limited to, inkjet printing, gravure printing, nozzle printing, letterpress printing, screen printing, dip coating, spin coating, doctor blade coating, roller printing, twist roller printing, flexographic lithographic printing, flexographic printing, rotary printing, spraying, brushing, pad printing, slot-type extrusion coating, and the like, wherein gravure printing, jet printing and inkjet are preferable.

The present application further relates to a light extraction layer material, which is selected from an organic compound or mixture or composition as described above.

The present application further relates to applications of the organic compound, mixture or composition as described above in an organic electronic device. The organic electronic device may be an organic light-emitting diode (OLED), an organic photovoltaic cell, an organic light-emitting cell, an organic field-effect transistor, an organic light-emitting field-effect transistor, an organic laser, an organic spin-electron device, an organic sensor, an organic plasma-emitting diode, and the like. Particularly preferred is OLED. Specific solutions are as follows:

An organic light-emitting device including two electrodes, one or more organic functional layers disposed between the two electrodes, and a light extraction layer disposed on a surface of one of the two electrodes and on a side away from the organic functional layer. The light extraction layer includes an arylamine organic compound has a structure represented by formula (1).

Further, an organic electronic device is provided, which includes a bottom electrode, at least one functional layer, a top electrode, and a light extraction layer that are stacked in sequence. The light extraction layer includes an arylamine organic compound described in the present application.

In an embodiment, the bottom electrode is an anode, the top electrode is a cathode, and the light extraction layer is located on the surface of the cathode.

In some embodiments, the organic electronic device according to the present application generally has a thickness of 10 nm to 200 nm, preferably 20 nm to 150 nm, more preferably 30 nm to 100 nm, and most preferably 40 nm to 90 nm.

In an embodiment, an organic electronic device according to the present application includes one or more organic functional layers, wherein the organic functional layers are selected from one or more layers of an electron injection layer, an electron transport layer, a hole injection layer, a hole transport layer, and a light-emitting layer, and wherein at least one light-emitting layer is included therein.

In some embodiments, the light-emitting layer in the organic electronic device according to the present application includes a light-emitting material selecting from a singlet emitter, a triplet emitter, or a TADF material.

In some embodiments, the organic functional layer in the organic electronic device according to the present application is selected from a hole transport layer, a light-emitting layer, and an electron transport layer.

In some embodiments, the organic functional layer in the organic electronic device according to the present application is selected from a hole injection layer, a hole transport layer, a light-emitting layer, an electron transport layer, and an electron injection layer.

Some descriptions of singlet emitters, triplet emitters, and TADF materials are described in detail in WO2017092619A.

Further, the organic functional layer in the organic electronic device according to the present application includes a light-emitting layer. Further, the light-emitting layer material is selected from triplet light-emitting materials.

The triplet light-emitting material has a structure represented by the following formula: 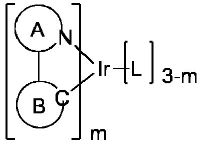
wherein m is selected from 1 or 2 or 3;
ring A is selected from a substituted or unsubstituted N-containing heteroaromatic group having 5 to 30 ring atoms;
ring B is selected from a substituted or unsubstituted aromatic or heteroaromatic group having 6 to 30 ring atoms;
L is a monovalent anionic organic ligand.

In an embodiment, ring A is selected from any one of the following groups: 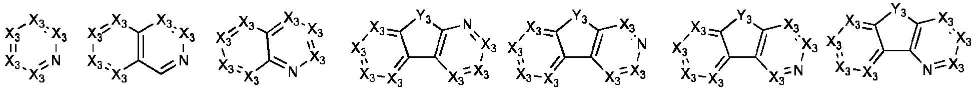 wherein N indicates a linkage site.

In an embodiment, ring B is selected from any one of the following groups: 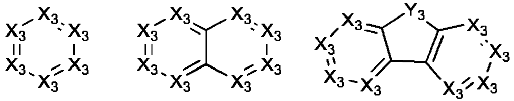 wherein X₃ is independently selected from CR₁₁ or N;
Y₃ is independently selected from CR₁₂R₁₃, NR₁₄, O, S, S=O, SO₂, PR₁₄, BR₁₄, SiR₁₂R₁₃;
R₁₁, R₁₂, R₁₃, R₁₄ are each independently selected from hydrogen, deuterium, a linear alkyl group having 1 to 20 carbon atoms, a branched or cyclic alkyl group having 3 to 20 carbon atoms, a cyano group, a nitro group, -CF₃, -OCF₃, -Cl, -Br, -F, a substituted or unsubstituted aromatic group having 6 to 30 ring atoms, a substituted or unsubstituted heteroaromatic group having 5 to 30 ring atoms, or a combination of thereof.

Further, the triplet light-emitting material is selected from the following formulas:

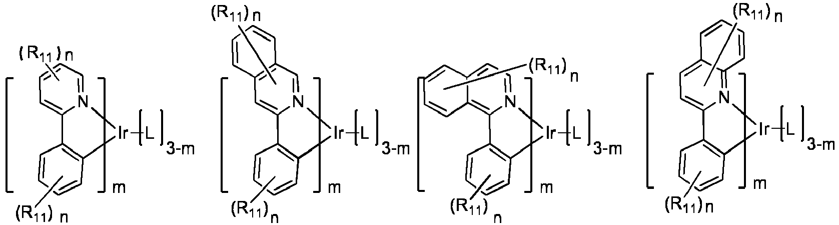

Further, R₁₁ is independently selected from hydrogen, deuterium, a linear alkyl group having 1 to 10 carbon atoms, a branched or cyclic alkyl group having 3 to 10 carbon atoms, a cyano group, a nitro group, -CF₃, -OCF₃, -Cl, -Br, -F, an aromatic group having 6 to 20 ring atoms, a heteroaromatic group having 5 to 20 ring atoms, or a combination of thereof;
n is independently selected from 0, 1, 2, 3, 4, or 5; and m is as described above.

In an embodiment, the triplet light-emitting material according to the present application is selected from the following structures:

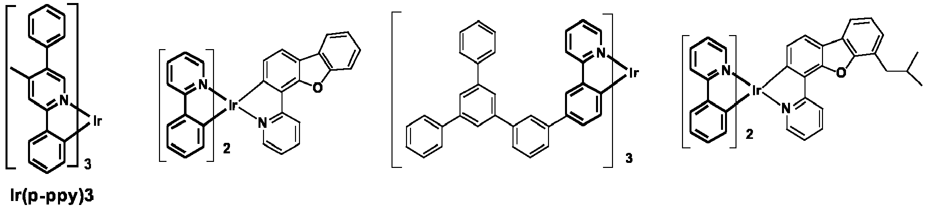

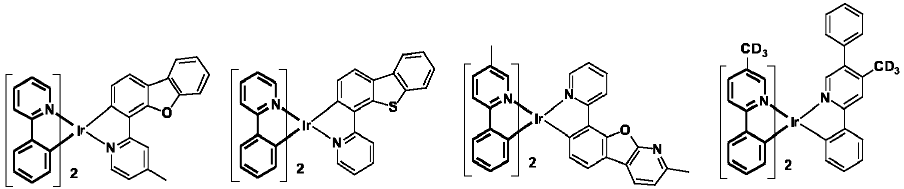

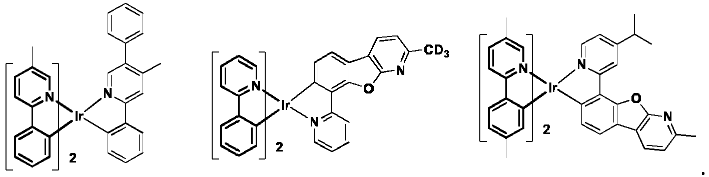

The organic electronic device according to the present application may be, but is not limited to, an organic light-emitting diode (OLED), an organic photovoltaic cell, an organic light-emitting cell, an organic field-effect transistor, an organic light-emitting field-effect transistor, an organic laser, an organic spin-electron device, an organic sensor, an organic plasma-emitting diode, and the like. Particularly preferred is OLED.

The cathode and anode in the device structure of the OLED are described below.

The anode may include conductive metals or metal oxides, or conductive polymers. The anode can be easily injected into the hole injection layer (HIL) or the hole transport layer (HTL) or the light-emitting layer. In an embodiment, the absolute value of the difference between the work function of the anode and HOMO level or the valence band level of the emitter in the light-emitting layer or the p-type semiconductor materials serving as HIL or HTL or EBL is less than 0.5 eV, preferably less than 0.3 eV, and more preferably less than 0.2 eV. Examples of the anode materials include, but are not limited to, Al, Cu, Au, Ag, Mg, Fe, Co, Ni, Mn, Pd, Pt, ITO, aluminum-doped zinc oxide (AZO), and the like. Other suitable anode materials are known and can be easily selected and used by those skilled in the art. The anode materials may be deposited using any suitable technique, such as a suitable physical vapor deposition method, including radio frequency magnetron sputtering, vacuum thermal evaporation, e-beam, and the like. In some embodiments, the anode is patterned. Patterned ITO conductive substrates are commercially available and can be used to prepare devices according to the present application.

The cathode may include conductive metals or metal oxides. The cathode can easily inject electrons into the EIL or ETL or directly into the light-emitting layer. In an embodiment, the absolute value of the difference between the work function of the cathode and the LUMO level or the conduction band level of the emitter in the light-emitting layer or the n-type semiconductor materials serving as the electron injection layer (EIL) or the electron transport layer (ETL) or the hole blocking layer (HBL) is less than 0.5 eV, preferably less than 0.3 eV, and more preferably less than 0.2 eV. In principle, all materials that can be used as cathodes for OLEDs are possible to be used as cathode materials for the devices of the present application. Examples of the cathode materials include, but are not limited to, Al, Au, Ag, Ca, Ba, Mg, LiF/Al, MgAg alloy, BaF₂/Al, Cu, Fe, Co, Ni, Mn, Pd, Pt, ITO, and the like. The cathode materials may be deposited using any suitable technique, such as a suitable physical vapor deposition method, including radio frequency magnetron sputtering, vacuum thermal evaporation, e-beam, and the like.

In an embodiment, in an organic light-emitting device according to the present application, in particular an organic light emitting diode, the light extraction layer is located on the surface of the cathode.

In some more preferred embodiments, the light extraction layer in the organic light-emitting device according to the present application generally has a thickness of 10 nm to 200 nm, preferably 20 nm to 150 nm, more preferably 30 nm to 100 nm, and most preferably 40 nm to 90 nm.

The present application also relates to applications of organic light-emitting devices according to the present application in various electronic devices, which may be, but are not limited to, display devices, lighting devices, light sources, sensors, and the like.

The present application will be specifically described by way of specific examples, which are only part examples of the present application and are not intended to limit the present application.

### Specific examples

### Example 1

Synthetic route of organic compound C1 in this example is as follows:

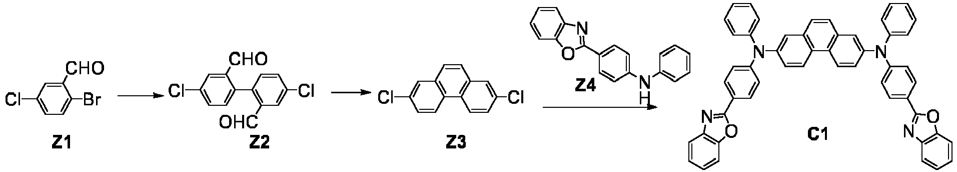

Compound Z1 (6.5 g, 30 mmol), bis(pinacolato)diboron (4.06 g, 16 mmol), palladium acetate (1 mmol), DPEPhos (bis(2-diphenylphosphine) phenyl ether, 2 mmol), and t-BuOK (potassium tert-butoxide, 130 mmol) were added to toluene, air was displaced with nitrogen, and the reaction was carried out under a temperature of 110 °C to obtain a reaction solution. After the reaction solution was cooled to room temperature, water was added for layering. The aqueous layer was extracted with ethyl acetate. The organic layers were combined, dried over anhydrous sodium sulfate, and concentrated, and then purified on a silica gel column to obtain a total of 3.15 g of compound Z2 with a yield of 76%.

Compound Z2 (2.5 g, 9 mmol) was added to acetic acid to obtain a mixture, the mixture was heated to a temperature of 100 °C, and 1 g of hydrazine hydrate and acetic acid were added, which were reacted until the reaction was completed as indicated by TLC. Then cooled to room temperature, added water, and extracted with dichloromethane. The organic phases were combined, and washed with deionized water. The organic layers were collected and then purified by column chromatography to obtain a total of 1.28 g white solid Z3 with a yield of 58%.

Compound Z3 (1.23 g, 5 mmol) and compound Z4 (3.14 g, 11 mmol) were dissolved in anhydrous toluene, and sodium tert-butoxide (0.96 g, 10 mmol) and tris(dibenzylideneacetone)dipalladium (0.14 g, 0.15 mmol) were added, after air was displaced with nitrogen for three times, tris-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 3.06 g of organic compound C1 with a yield of 82%. MS: 746[M+].

### Example 2

Synthetic route of organic compound C3 in this example is as follows:

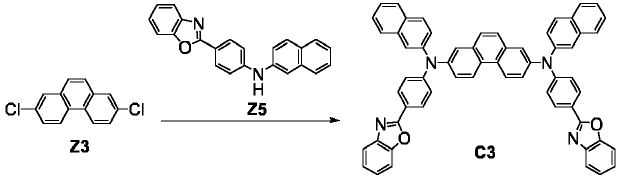

Compound Z3 (1.23 g, 5 mmol) and compound Z5 (3.7 g, 11 mmol) were dissolved in anhydrous toluene, and sodium tert-butoxide (0.96 g, 10 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 3.21 g of organic compound C3 with a yield of 76%. MS: 846[M+].

### Example 3

Synthetic route of organic compound C6 in this example is as follows:

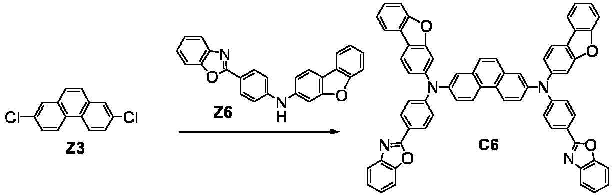

Compound Z3 (1.23 g, 5 mmol) and compound Z6 (4.13 g, 11 mmol) were dissolved in anhydrous toluene, and sodium tert-butoxide (0.96 g, 10 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 3.28 g of organic compound C6 with a yield of 71%. MS: 926[M+].

### Example 4

Synthetic route of organic compound C21 in this example is as follows:

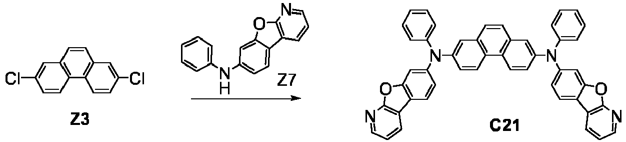

Compound Z3 (1.23 g, 5 mmol) and compound Z7 (2.86 g, 11 mmol) were dissolved in anhydrous toluene, and sodium tert-butoxide (0.96 g, 10 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 2.84 g of organic compound C21 with a yield of 82%. MS (ASAP) = 694[M+].

### Example 5

Synthetic route of organic compound C26 in this example is as follows:

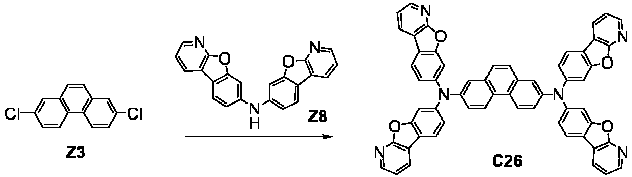

Compound Z3 (1.23 g, 5 mmol) and compound Z8 (3.86 g, 11 mmol) were dissolved in anhydrous toluene, and sodium tert-butoxide (0.96 g, 10 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 2.54 g of organic compound C26 with a yield of 58%. MS:876[M+].

### Example 6

Synthetic route of organic compound C69 in this example is as follows:

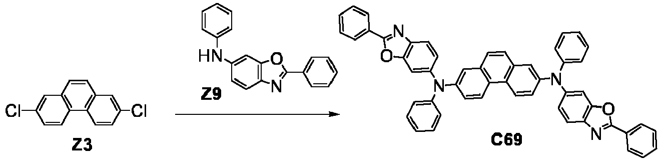

Compound Z3 (1.23 g, 5 mmol) and compound Z9 (3.14 g, 11 mmol) were dissolved in anhydrous toluene, and sodium tert-butoxide (0.96 g, 10 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 2.87 g of organic compound C69 with a yield of 77%. MS: 746[M+].

### Example 7

Synthetic route of organic compound in this example starting from C57 is as follows:

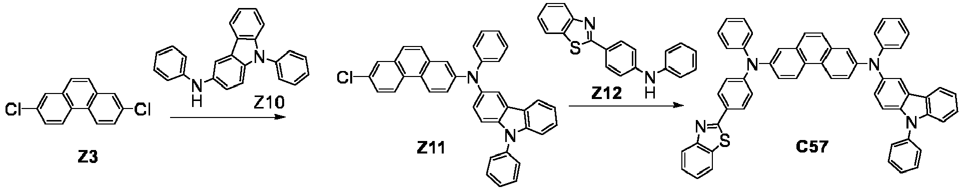

Compound Z3 (2.46 g, 10 mmol) and compound Z10 (3.67 g, 11 mmol) were dissolved in anhydrous toluene, and sodium tert-butoxide (1.15 g, 12 mmol) and tris(dibenzylideneacetone)dipalladium (0.27 g, 0.3 mmol) were added, after air was displaced with nitrogen for three times, tri-tert-butylphosphine (0.3 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 3.91 g of compound Z11 with a yield of 72%.

Compound Z11 (2.72 g, 5 mmol) and compound Z12 (1.81 g, 6 mmol) were dissolved in anhydrous toluene, and sodium tert-butoxide (0.57 g, 6 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 3.36 g of organic compound C57 with a yield of 83%. MS: 810[M+].

### Example 8

Synthetic route of organic compound C39 in this example is as follows:

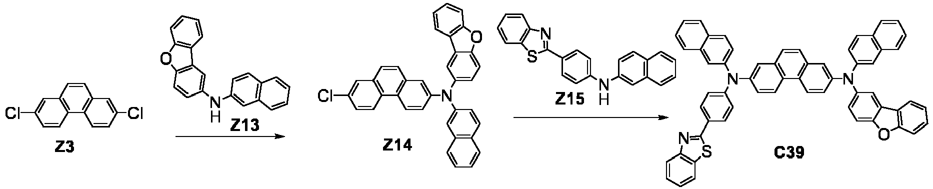

Compound Z3 (2.46 g, 10 mmol) and compound Z13 (3.4 g, 11 mmol) were dissolved in anhydrous toluene, sodium tert-butoxide (1.15 g, 12 mmol) and tris(dibenzylideneacetone)dipalladium (0.27 g, 0.3 mmol) were added, after air was displaced with nitrogen for three times, tri-tert-butylphosphine (0.3 mmol) was added, which were gradually heated to a temperature of 80 °C to carried out a reaction under stirring for 12 hours, and the heat source was removed. After the system was cooled, organic layer was separated by adding deionized water, and extracted three times with ethyl acetate, concentrated under reduced pressure, and purified on a silica gel column to obtain 3.58 g of compound Z14 with a yield of 69%.

Compound Z14 (2.6 g, 5 mmol) and compound Z15 (2.11 g, 6 mmol) were dissolved in anhydrous toluene, and sodium tert-butoxide (0.57 g, 6 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 3.13 g of organic compound C39 with a yield of 75%. MS: 835[M+].

### Example 9

Synthetic route of organic compound C17 in this example is as follows:

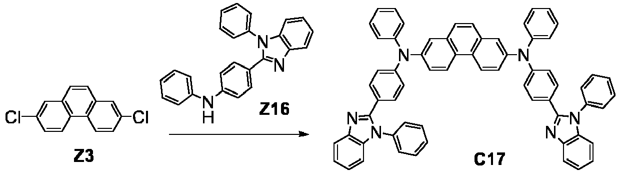

Compound Z3 (1.23 g, 5 mmol) and compound Z16 (3.97 g, 11 mmol) were dissolved in anhydrous toluene, and sodium tert-butoxide (0.96 g, 10 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 3.18 g of organic compound C17 with a yield of 71%. MS:896[M+].

### Example 10

Synthetic route of organic compound C11 in this example is as follows:

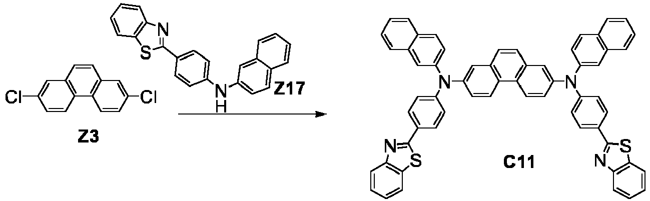

Compound Z3 (1.23 g, 5 mmol) and compound Z17 (3.87 g, 11 mmol) were dissolved in anhydrous toluene, and sodium tert-butoxide (0.96 g, 10 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 2.81 g of organic compound C11 with a yield of 64%. MS: 878[M+].

### Example 11

Synthetic route of organic compound C158 in this example is as follows:

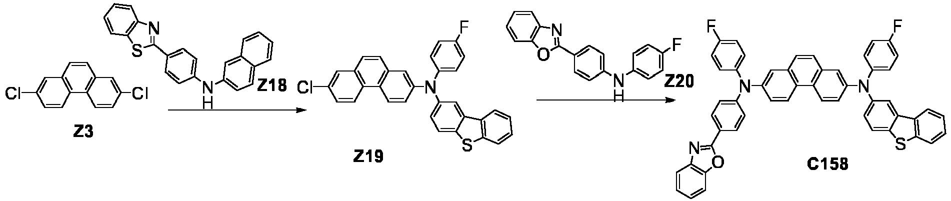

Compound Z3 (2.46 g, 10 mmol) and compound Z18 (3.87 g, 11 mmol) were dissolved in anhydrous toluene, and sodium tert-butoxide (1.15 g, 12 mmol) and tris(dibenzylideneacetone)dipalladium (0.27 g, 0.3 mmol) were added, after air was displaced with nitrogen for three times, tri-tert-butylphosphine (0.3 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 2.66 g of compound Z19 with a yield of 53%.

Compound Z19 (2.51 g, 5 mmol) and compound Z20 (1.82 g, 6 mmol) were dissolved in anhydrous toluene, and sodium tert-butoxide (0.57 g, 6 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, and the heat source was removed. After the system was cooled, organic layer was separated by adding deionized water, and extracted three times with ethyl acetate, concentrated under reduced pressure, and purified on a silica gel column to obtain 2.58 g of organic compound C158 with a yield of 67%. MS: 771 [M+].

### Example 12

Synthetic route of organic compound C80 in this example is as follows:

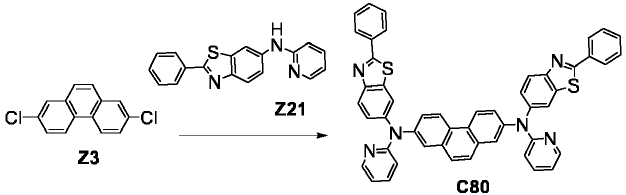

Compound Z3 (1.23 g, 5 mmol) and compound Z21 (3.33 g, 11 mmol) were dissolved in anhydrous toluene, and sodium tert-butoxide (0.96 g, 10 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 1.75 g of organic compound C80 with a yield of 45%. MS: 780[M+].

### Example 13

Synthetic route of organic compound C89 in this example is as follows:

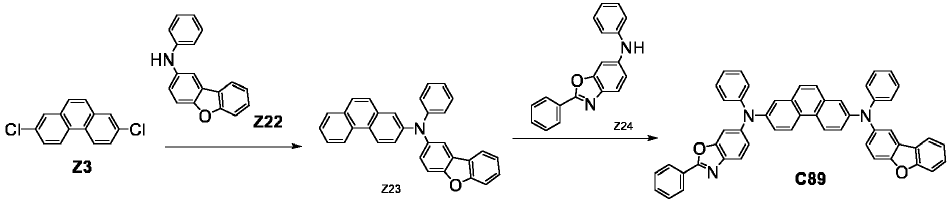

Compounds Z3 (2.46 g, 10 mmol) and Z22 (2.85 g, 11 mmol) were dissolved in anhydrous toluene, and sodium tert-butoxide (1.15 g, 12 mmol) and tris(dibenzylideneacetone)dipalladium (0.27 g, 0.3 mmol) were added, after air was displaced with nitrogen for three times, tri-tert-butylphosphine (0.3 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 2.65 g of compound Z23 with a yield of 61%.

Compound Z23 (2.17 g, 5 mmol) and compound Z24 (1.72 g, 6 mmol) were dissolved in anhydrous toluene, and sodium tert-butoxide (0.57 g, 6 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 2.59 g of organic compound C89 with a yield of 72%. MS: 719[M+].

### Example 14

Synthetic route of organic compound C196 in this example is as follows:

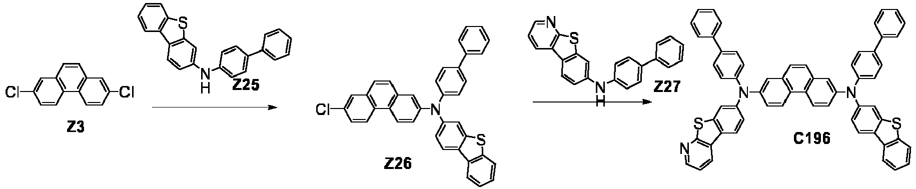

Compound Z3 (2.46 g, 10 mmol) and compound Z25 (3.86 g, 11 mmol) were dissolved in anhydrous toluene, and sodium tert-butoxide (1.15 g, 12 mmol) and tris(dibenzylideneacetone)dipalladium (0.27 g, 0.3 mmol) were added, after air was displaced with nitrogen for three times, tri-tert-butylphosphine (0.3 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 3.65 g of compound Z26 with a yield of 65%.

Compound Z26 (2.8 g, 5 mmol) and compound Z27 (2.11 g, 6 mmol) were dissolved in anhydrous toluene, and sodium tert-butoxide (0.57 g, 6 mmol) and tris(dibenzylideneacetone)dipalladium (0.14 g, 0.15 mmol) were added, after air was displaced with nitrogen for three times, tri-tert-butylphosphine (0.15 mmol) was added, which were gradually heated to a temperature of 80 °C to carried out a reaction under stirring for 12 hours, then the heat source was removed. After the system was cooled, organic layer was separated by adding deionized water, and extracted three times with ethyl acetate, concentrated under reduced pressure, and purified on a silica gel column to obtain 2.5 g of organic compound C196 with a yield of 57%. MS: 877[M+].

### Comparative example

### The organic compound in this comparative example was CBP (4,4'-bis(9-carbazole) biphenyl).

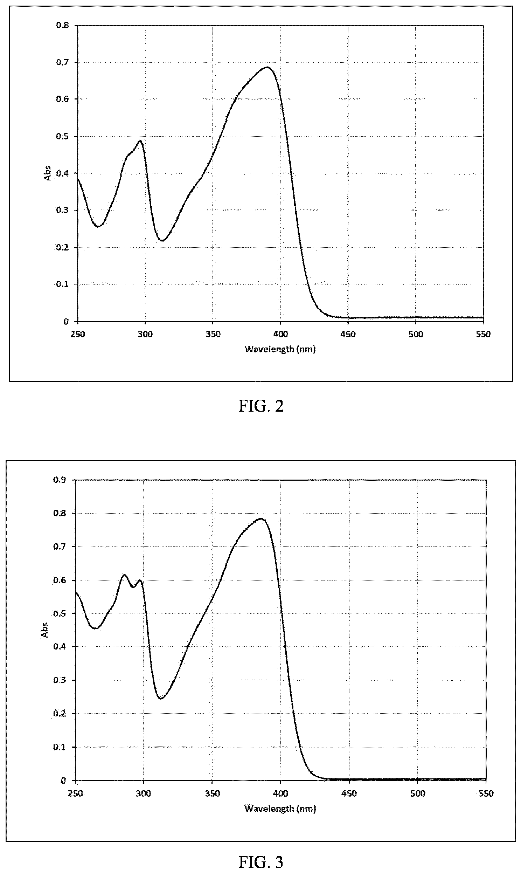

The UV-visible absorption spectra of organic compound C1 of Example 1 and organic compound C21 of Example 4 in dichloromethane were tested. The UV-visible absorption spectrum of the organic compound C1 of Example 1 in the solution is shown in FIG. 2, and the UV-visible absorption spectrum of the organic compound C21 of Example 4 in the solution is shown in FIG. 3.

### Extinction coefficient and refractive index test

The organic compound of Examples 1 to 14 and Comparative Example were evaporated on single crystal silicon by vacuum evaporation to form a thin film of 50 nm. The single crystal silicon was placed on the sample stage of an ellipsometer (ES-01), the incident angle was 70 degrees, and the test environment was atmospheric environment. The test results such as extinction coefficient k (extinction coefficient @430 nm) of the organic compounds for light with a wavelength of 430 nm and the refractive index n (refractive index @630 nm) of the organic compounds for light with a wavelength of 630 nm were fitted by the ellipsometer. The test results are shown in Table 1 below.

**Table 1:**

| | Organic compound | Extinction coefficient @430 nm | Refractive index @630 nm |
|---|---|---|---|
| Example 1 | C1 | 0.01 | 1.90 |
| Example 2 | C3 | 0.02 | 1.91 |
| Example 3 | C6 | 0.01 | 1.92 |
| Example 4 | C21 | 0.01 | 1.87 |
| Example 5 | C26 | 0.01 | 1.89 |
| Example 6 | C69 | 0.03 | 1.91 |
| Example 7 | C57 | 0.03 | 1.87 |
| Example 8 | C39 | 0.04 | 1.88 |
| Example 9 | C17 | 0.05 | 1.91 |
| Example 10 | C11 | 0.05 | 1.92 |
| Example 11 | C158 | 0.03 | 1.90 |
| Example 12 | C80 | 0.04 | 1.92 |
| Example 13 | C89 | 0.02 | 1.89 |
| Example 14 | C196 | 0.01 | 1.88 |
| Comparative example | CBP | 0 | 1.74 |

It can be seen from FIGs. 2 and 3 and Table 1 that:
Compared with the organic compounds in the comparative examples, the arylamine organic compounds in Examples 1 to 14 have weaker light absorption in the visible light band and higher absorption of light in the ultraviolet band.

Compared with the organic compounds in the comparative examples, the arylamine organic compounds of Examples 1 to 14 have higher refractive index.

Preparation of OLED devices and performance test thereof
The preparation process of the OLED devices using the above-mentioned materials will be described in detail by means of specific examples. The OLED devices have structures as follows: ITO/Ag/ITO (anode)/HATCN/SFNFB/m-CP: Ir(p-ppy)₃/NaTzF₂/LiF/Mg: Ag/light extraction layer.

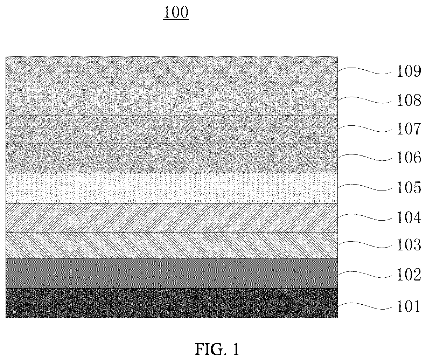

The schematic diagram of an OLED device is shown in FIG. 1, wherein 101 is a substrate, 102 is an anode, 103 is a HIL, 104 is a HTL, 105 is a light-emitting layer, 106 is an electron transport layer (ETL), 107 is an electron injection layer, 108 is a cathode, and 109 is a light extraction layer.

The preparation process was as follows:
Cleaning the ITO/Ag/ITO conductive glass anode layer, followed by ultrasonic cleaning with deionized water, acetone, and isopropanol for 15 minutes, and then treating in a plasma cleaner for 5 minutes to improve the work function of the electrode. Forming a hole injection layer by subjecting a hole injection layer material HATCN to evaporation through vacuum evaporation on the ITO anode layer at an evaporation rate of 1 Å/s, which has a thickness of 5 nm. Forming a hole transport layer by subjecting the hole transport material SFNFB to evaporation through vacuum evaporation on the hole injection layer, which has a thickness of 80 nm. Forming a light-emitting layer by subjecting a light-emitting layer to evaporation on the hole transport layer, wherein m-CP is used as a host material, Ir(p-ppy)₃ is used as a doping material, and the mass ratio of Ir(p-ppy)₃ and m-CP is 1:9, it has a thickness of 30 nm. Forming an electron transport layer by subjecting an electron transport material NaTzF₂ to evaporation through vacuum evaporation on the light-emitting layer, which has a thickness of 30 nm. Forming an electron injection layer by subjecting an electron injection layer LiF to vacuum evaporation on the electron transport layer, which has a thickness of 1 nm. Forming a cathode layer by subjecting a cathode Mg: Ag (with a Mg: Ag doping ratio of 9:1) layer to vacuum evaporation on the electron injection layer, which has a thickness of 15 nm. Forming a light extraction layer by subjecting a light extraction layer organic compound to vacuum evaporation on the cathode layer, which has a thickness of 60 nm.

Device Example 1: the light extraction layer organic compound of the OLED device is C1.

Device Example 2: the light extraction layer organic compound of the OLED device is C3.

Device Example 3: the light extraction layer organic compound of the OLED device is C6.

Device Example 4: the light extraction layer organic compound of the OLED device is C21.

Device Example 5: the light extraction layer organic compound of the OLED device is C26.

Device Example 6: the light extraction layer organic compound of the OLED device is C69.

Device Example 7: the light extraction layer organic compound of the OLED device is C57.

Device Example 8: the light extraction layer organic compound of the OLED device is C39.

Device Example 9: The light extraction layer organic compound of the OLED device was C17.

Device Example 10: the light extraction layer organic compound of the OLED device is C11.

Device Example 11: the light extraction layer organic compound of the OLED device is C158.

Device Example 12: the light extraction layer organic compound of the OLED device is C80.

Device Example 13: the light extraction layer organic compound of the OLED device is C89.

Device Example 14: the light extraction layer organic compound of the OLED device is C196.

Device Comparative Example: the light extraction layer organic compound of the OLED device is CBP.

The structures of the compounds involved in the OLED device are as follows:

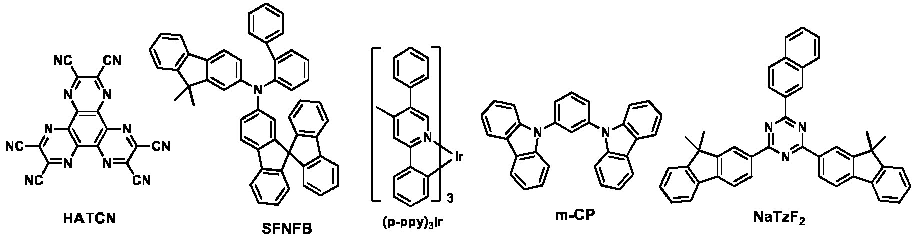

The luminous efficiency of the OLED devices in each of the Examples and Comparative Examples at a current density of 10 mA/cm² was measured using an EQE optical test instrument, and the results are shown in Table 2 below.

**Table 2:**

| OLED device | Light extraction layer compound | Luminous efficiency |
|---|---|---|
| Device Example 1 | C1 | 1.23 |
| Device Example 2 | C3 | 1.22 |
| Device Example 3 | C6 | 1.24 |
| Device Example 4 | C21 | 1.15 |
| Device Example 5 | C26 | 1.19 |
| Device Example 6 | C69 | 1.22 |
| Device Example 7 | C57 | 1.16 |
| Device Example 8 | C39 | 1.18 |
| Device Example 9 | C 17 | 1.21 |
| Device Example 10 | C11 | 1.25 |
| Device Example 11 | C158 | 1.19 |
| Device Example 12 | C80 | 1.23 |
| Device Example 13 | C89 | 1.20 |
| Device Example 14 | C196 | 1.17 |
| Device comparative | CBP | 1 |

As can be seen from Table 2, compared with the OLED devices in the comparative example, the OLED devices in Examples 1 to 14 have higher luminous efficiency. It can be seen that the organic compounds according to the present application can effectively improve the luminous efficiency of organic electronic devices.

The arylamine organic compounds of the present application contain phenanthrene, diarylamine, and fused heterocyclic ring in the structures, and have high glass temperature and high thermal stability. In addition, the arylamine organic compounds have high extinction coefficient for light in the ultraviolet band (i.e., ultraviolet light), and when the arylamine organic compounds are used as a light extraction layer of an organic electronic device, adverse effects of harmful light on electrodes and internal functional layers of the organic electronic device can be effectively avoided. In addition, the arylamine organic compound has a relatively small extinction coefficient for visible light and a relatively high transmittance for visible light, thereby reducing the influence of the light extraction layer on the luminous efficiency of the organic electronic device. Further, the arylamine organic compounds have high refractive index and can effectively reflect as much visible light as possible. Therefore, the arylamine organic compounds are used as light extraction layer materials in an organic electronic device, and the luminous efficiency of visible light of the organic electronic device can be effectively improved.

In view of the foregoing, the arylamine organic compounds, mixtures, compositions, and organic electronic devices provided in examples of the present application have been described in detail above, and the principles and embodiments of the present application are described by using specific examples herein. Descriptions of the above examples are merely intended to help understand the methods and core ideas of the present application. Meanwhile, for a person with ordinary skill in the art, there will be changes in the specific embodiments and application scopes based on the ideas of the present application. In view of the foregoing, the contents of the specification should not be construed as limiting the present application.

## Claims

1. An arylamine organic compound having a structure represented by formula (1): 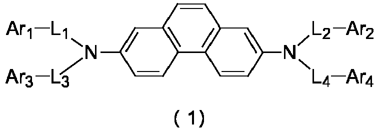 wherein:
L₁ to L₄ are each independently selected from a single bond, a substituted or unsubstituted aromatic group having 6 to 30 ring atoms, or a substituted or unsubstituted heteroaromatic group having 5 to 30 ring atoms;
Ar₁ is selected from any structure represented by formula (A-1) or formula (A-2);
Ar₂ is selected from any structure represented by formulas (A-2) to (A-6);
Ar₃ and Ar₄ are each independently selected from any structure represented by formulas (A-3) to (A-6) described above;
structures represented by formulas (A-1) to (A-6) are as follows: 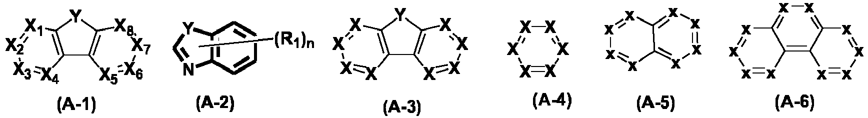
n is selected from 0, 1, 2, 3 or 4;
Y is selected from O, S or NR₂;
X and X₁ to X₈ are each independently selected from CR₃ or N, respectively, and at least one of X₁ to X₈ is selected from N; and
R₁, R₂ and R₃ are each independently selected from -H, -D, a linear alkyl group having 1 to 20 carbon atoms, a linear alkoxy group having 1 to 20 carbon atoms, a branched alkyl group having 3 to 20 carbon atoms, a branched alkoxy group having 3 to 20 carbon atoms, a cyclic alkyl group having 3 to 20 carbon atoms, a cyclic alkoxy group having 3 to 20 carbon atoms, a substituted or unsubstituted silyl group, a cyano group, an isocyano group, a hydroxyl group, a nitro group, an amino group, -CF₃, -Cl, -Br, -F, -I, an alkenyl group having 2 to 20 carbon atoms, or a substituted or unsubstituted aromatic group having 6 to 20 ring atoms, or a combination thereof.

2. The arylamine organic compound according to claim 1, wherein the formula (1) is selected from any structure represented by formulas (2-1) to (2-6):

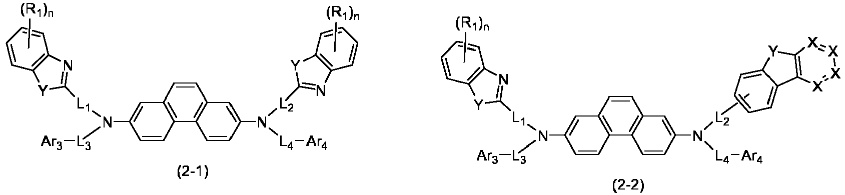

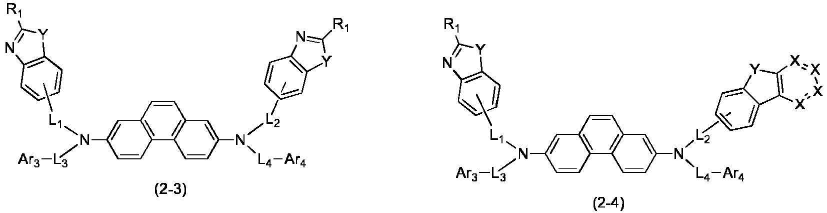

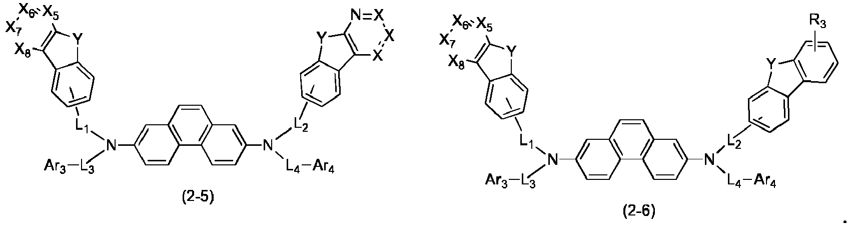

3. The arylamine organic compound according to claim 1, wherein the L₁ to L₄ are each independently selected from a single bond or any one of the following groups: 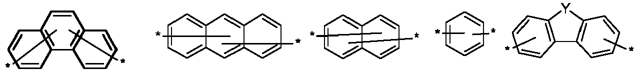 wherein: * indicates a linkage site.

4. The arylamine organic compound according to claim 1, wherein the formula (1) is selected from any structure represented by formulas (3-1) to (3-6):

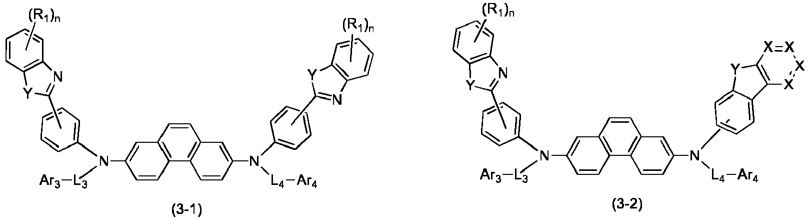

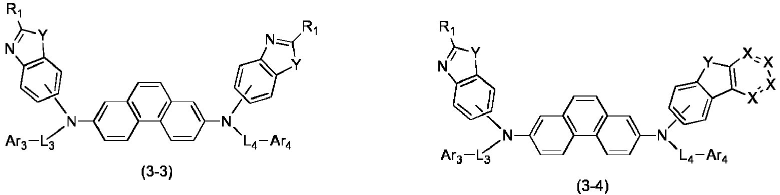

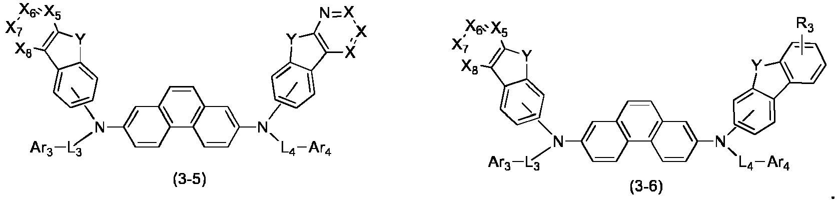

5. The arylamine organic compound according to claim 1, wherein the Ar₃ and the Ar₄ are each independently selected from any group represented by formulas (B-1) to (B-7): 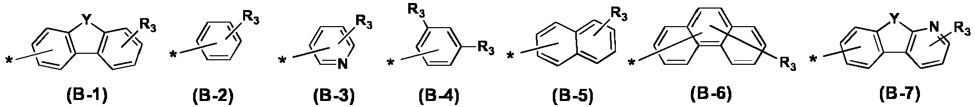 wherein:
* indicates a linkage site; and
R₃ is independently selected from -H, -D, a linear alkyl group having 1 to 10 carbon atoms, a branched alkyl group having 3 to 10 carbon atoms, a cyclic alkyl group having 3 to 10 carbon atoms, a silyl group, a cyano group, an isocyano group, a hydroxyl group, a nitro group, -CF₃, -Cl, -Br, -F, -I, phenyl, biphenyl, terphenyl, or naphthyl.

6. The arylamine organic compound according to claim 1, wherein *-L₃-Ar₃ and/or *-L₄-Ar₄ in the formula (1) are/is selected from the following groups: 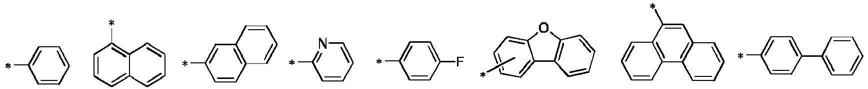 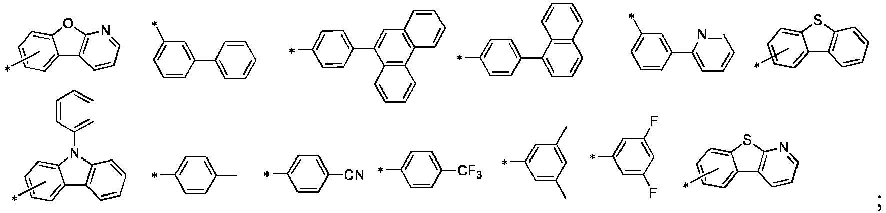 wherein: * indicates a linkage site.

7. The arylamine organic compound according to claim 1, wherein the arylamine organic compound is selected from any one of the following compounds:

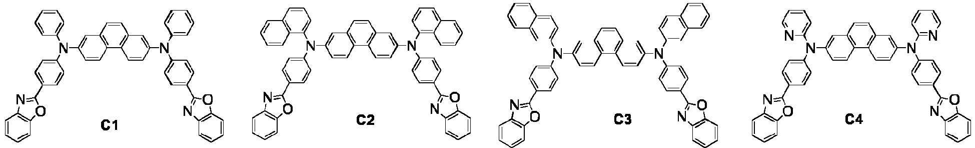

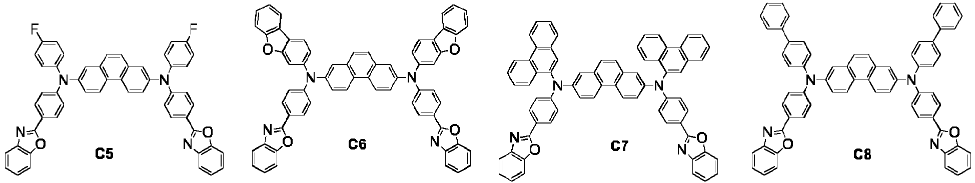

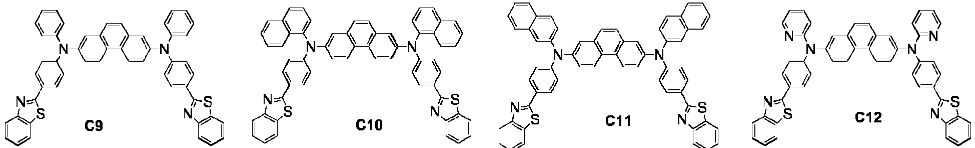

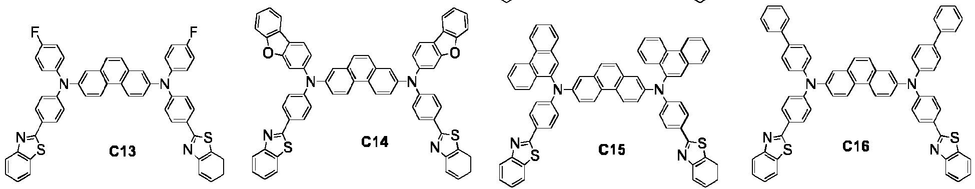

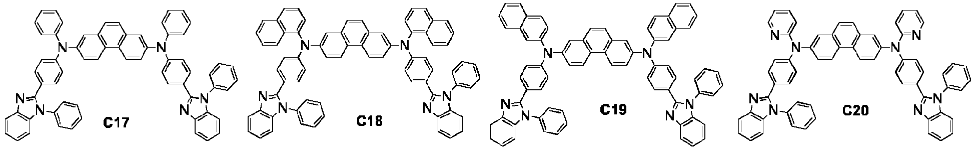

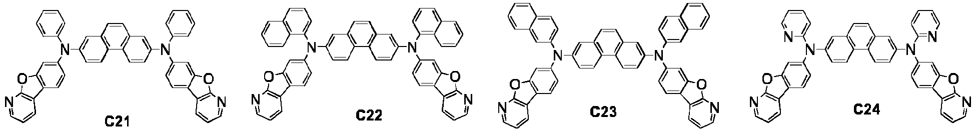

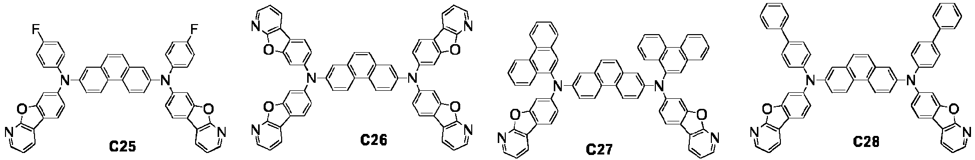

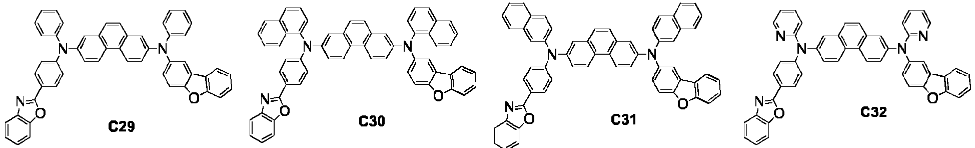

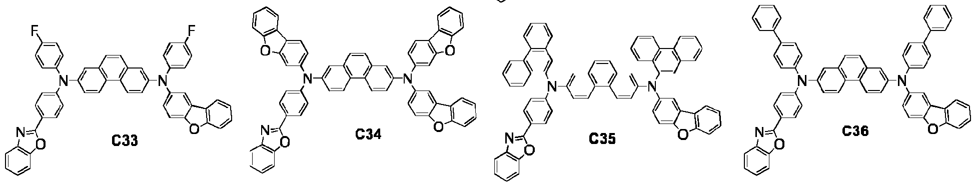

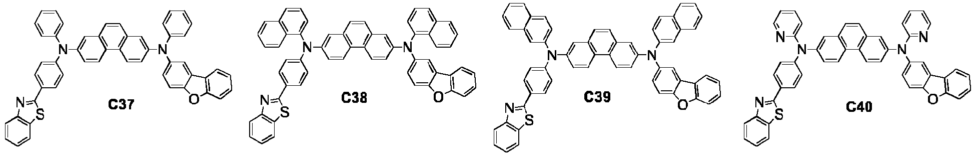

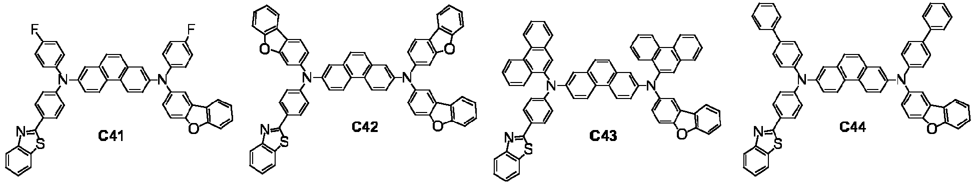

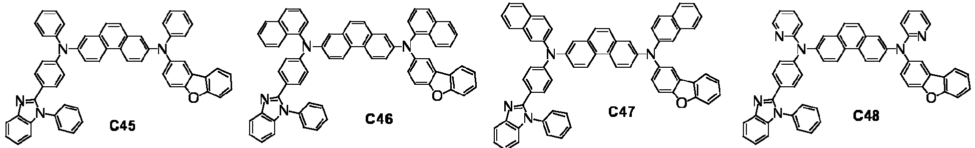

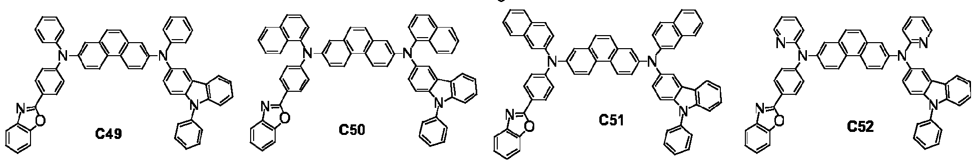

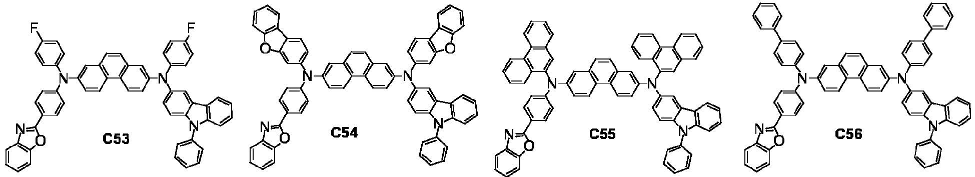

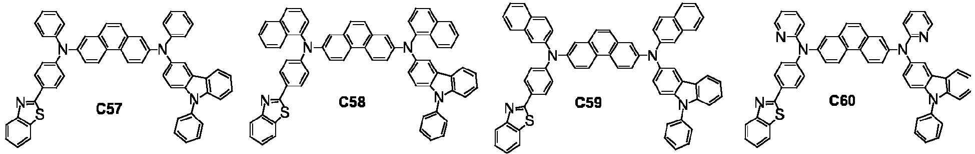

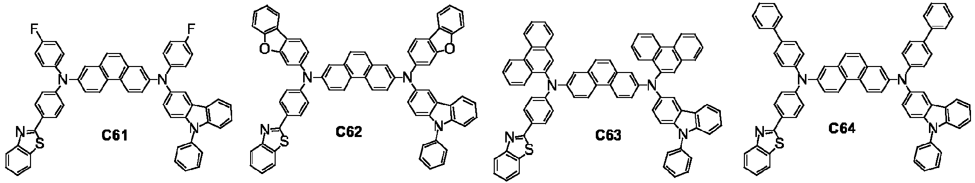

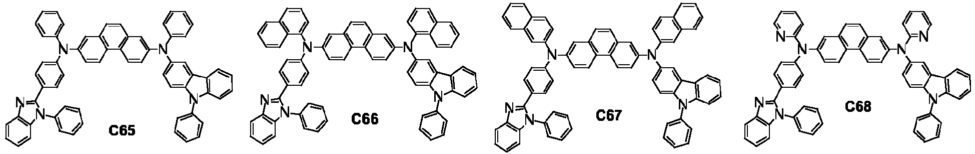

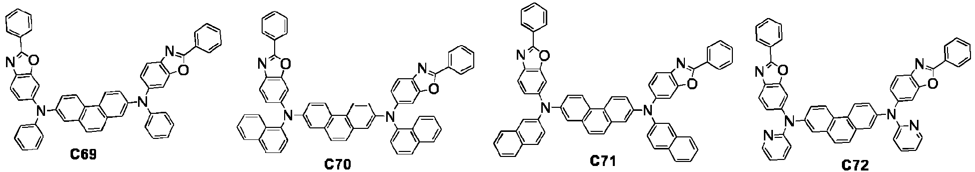

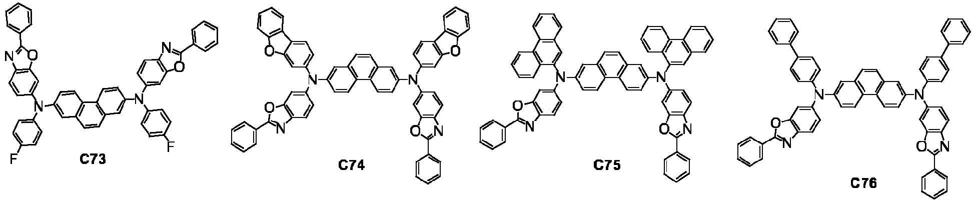

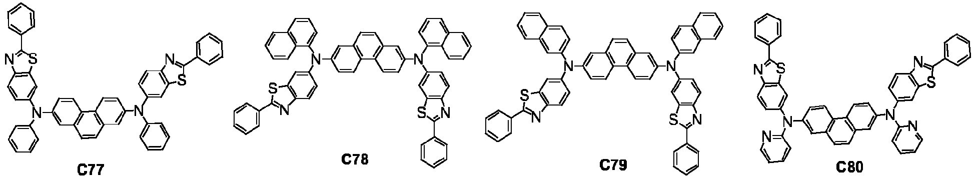

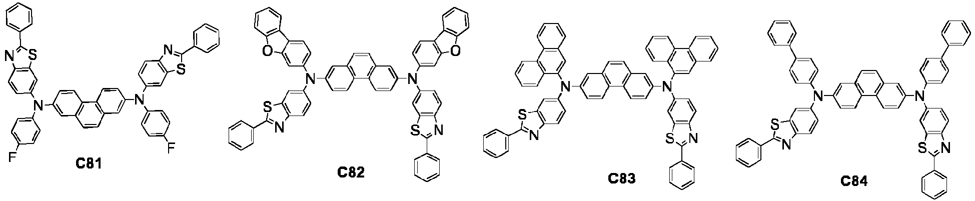

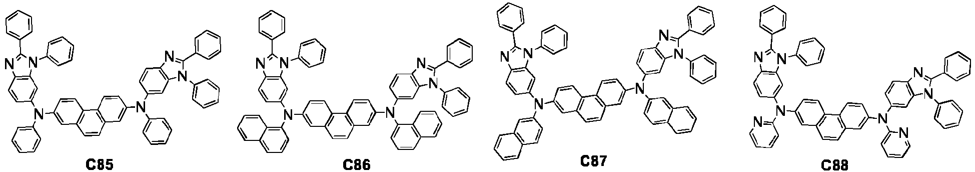

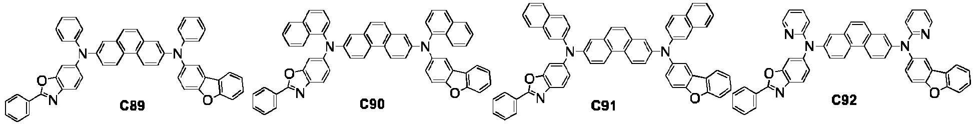

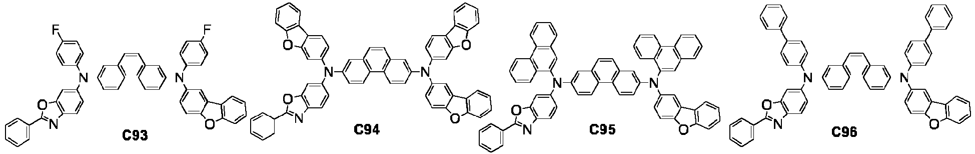

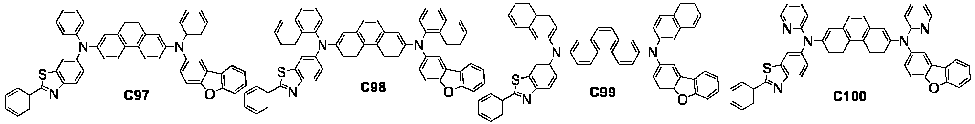

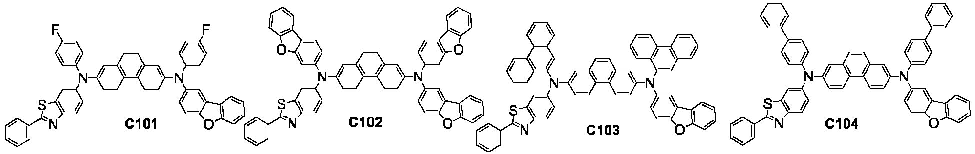

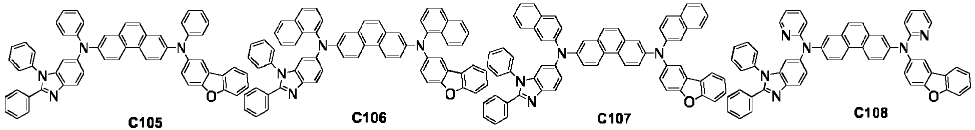

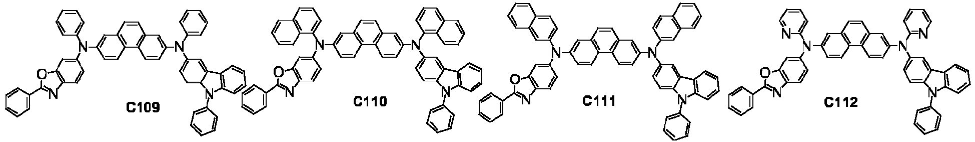

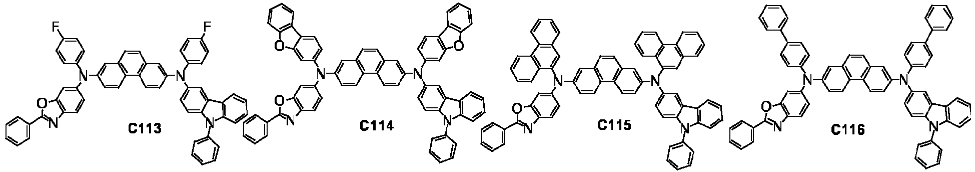

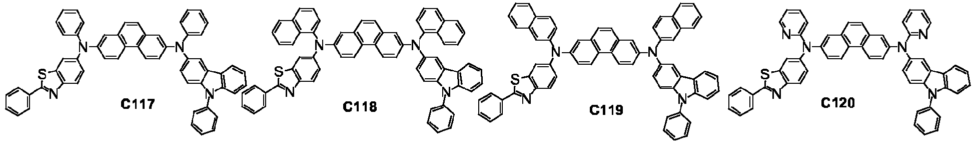

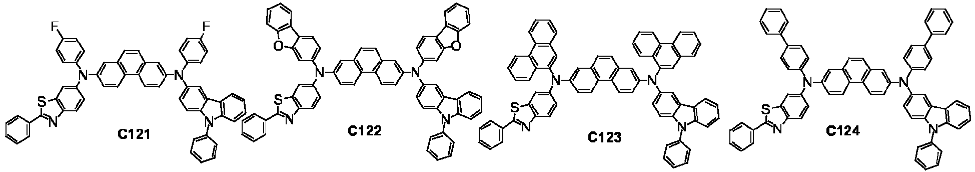

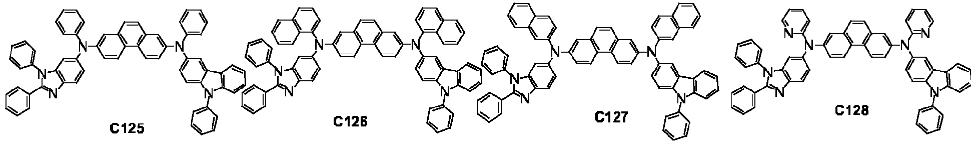

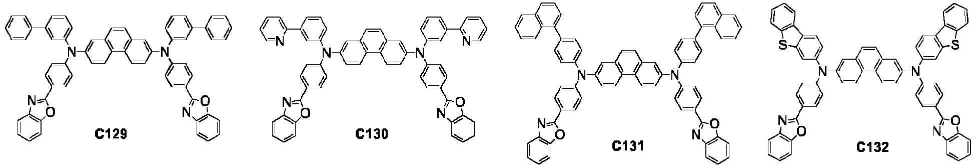

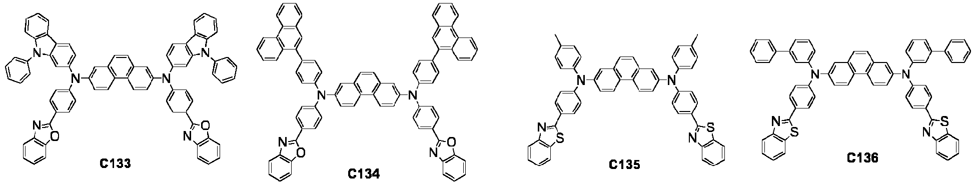

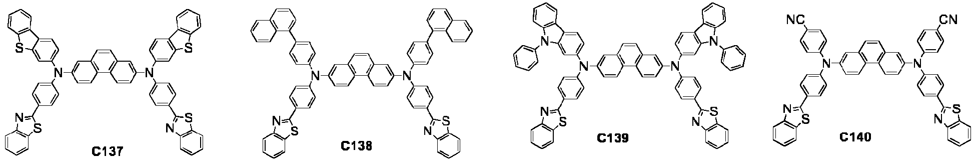

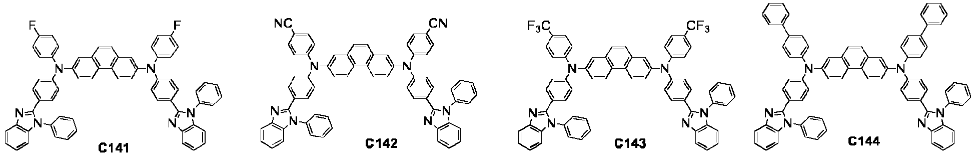

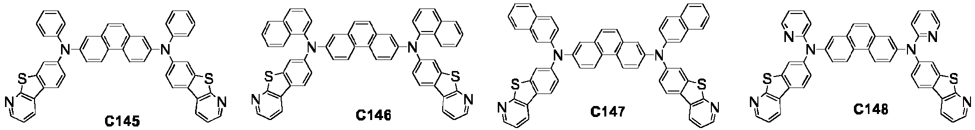

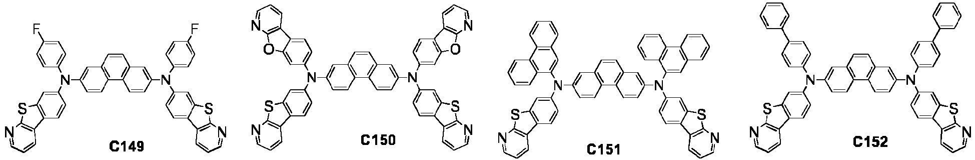

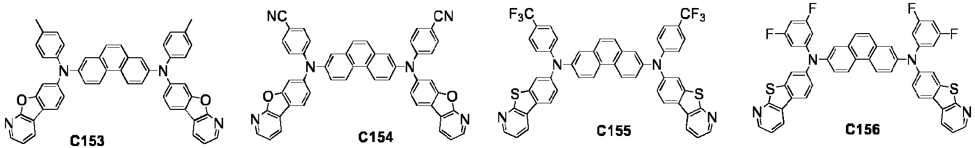

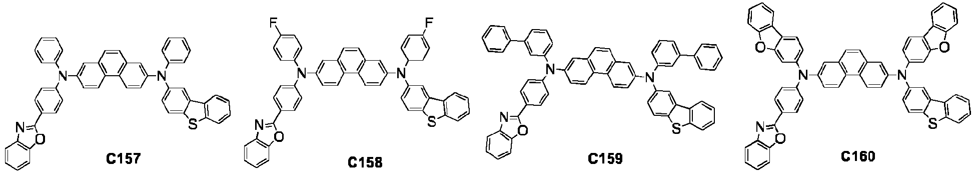

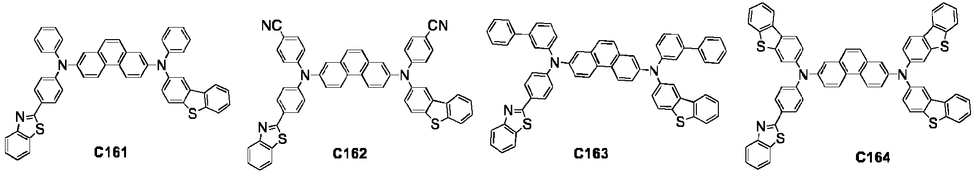

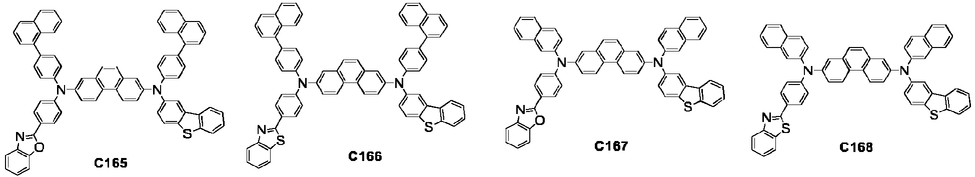

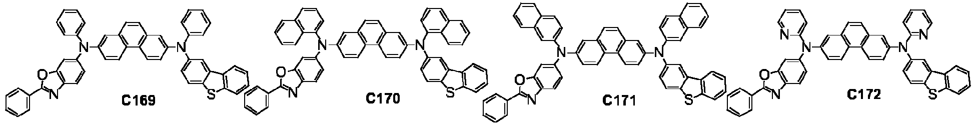

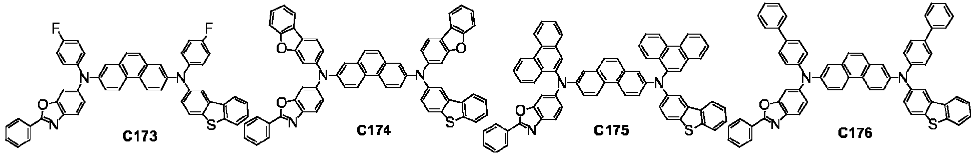

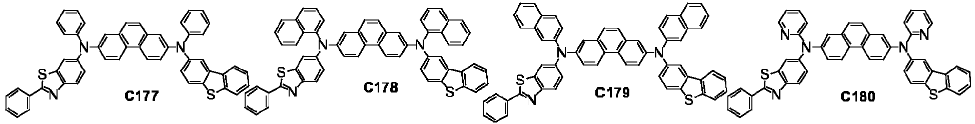

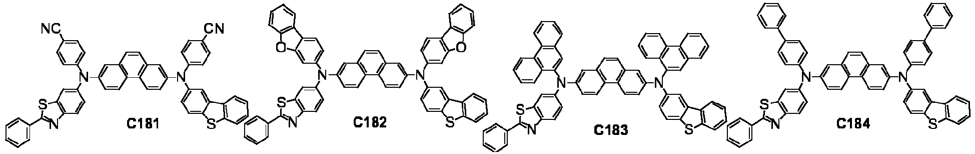

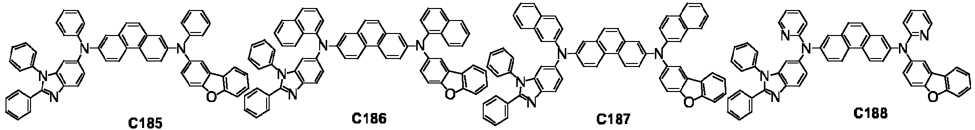

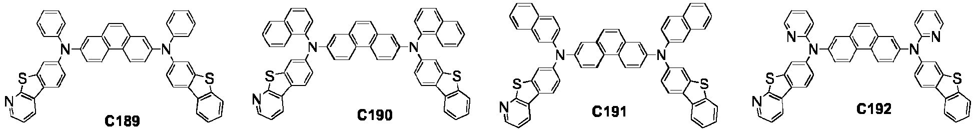

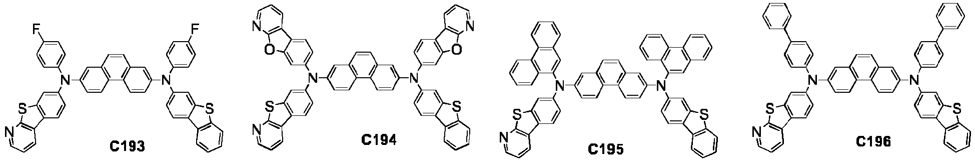

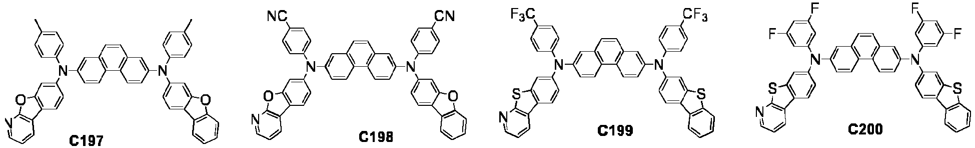

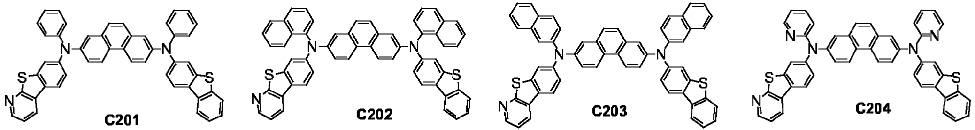

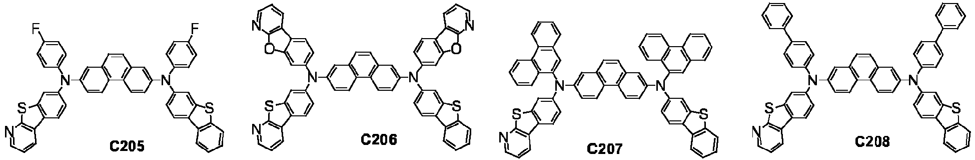

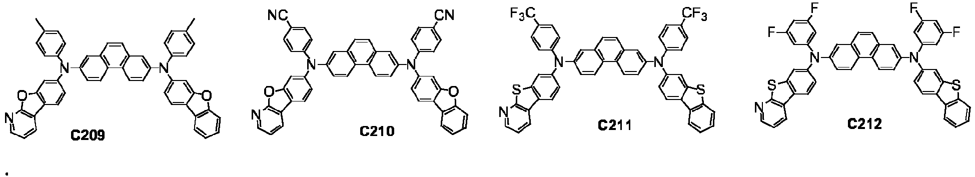

8. A mixture comprising an arylamine organic compound and at least one organic functional material, the at least one organic functional material is selected from a hole injection material, a hole transport material, an electron transport material, an electron injection material, an electron blocking material, a hole blocking material, a light-emitting material, a host material, a host material or an organic dye; wherein the arylamine organic compound has a structure represented by formula (1): 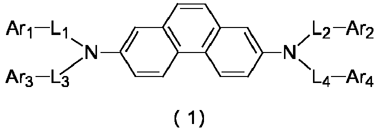 wherein:
L₁ to L₄ are each independently selected from a single bond, a substituted or unsubstituted aromatic group having 6 to 30 ring atoms, or a substituted or unsubstituted heteroaromatic group having 5 to 30 ring atoms;
An is selected from any structure represented by formula (A-1) or formula (A-2);
Ar₂ is selected from any structure represented by formulas (A-2) to (A-6);
Ar₃ and Ar₄ are each independently selected from any structure represented by formulas (A-3) to (A-6) described above;
structures represented by formulas (A-1) to (A-6) are as follows: 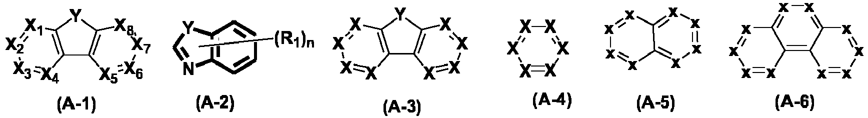
n is selected from 0, 1, 2, 3 or 4;
Y is selected from O, S or NR₂;
X and X₁ to X₈ are each independently selected from CR₃ or N, respectively, and at least one of X₁ to X₈ is selected from N; and
R₁, R₂ and R₃ are each independently selected from -H, -D, a linear alkyl group having 1 to 20 carbon atoms, a linear alkoxy group having 1 to 20 carbon atoms, a branched alkyl group having 3 to 20 carbon atoms, a branched alkoxy group having 3 to 20 carbon atoms, a cyclic alkyl group having 3 to 20 carbon atoms, a cyclic alkoxy group having 3 to 20 carbon atoms, a substituted or unsubstituted silyl group, a cyano group, an isocyano group, a hydroxyl group, a nitro group, an amino group, -CF₃, -Cl, -Br, -F, -I, an alkenyl group having 2 to 20 carbon atoms, or a substituted or unsubstituted aromatic group having 6 to 20 ring atoms, or a combination thereof.

9. The mixture according to claim 8, wherein the formula (1) is selected from any structure represented by formulas (2-1) to (2-6):

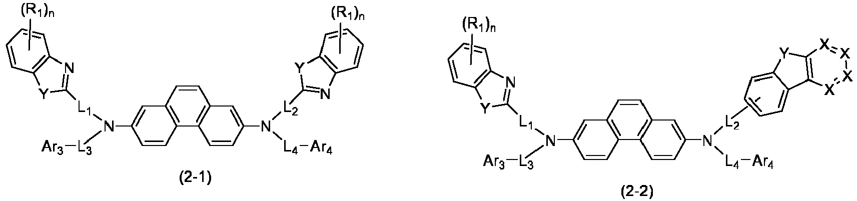

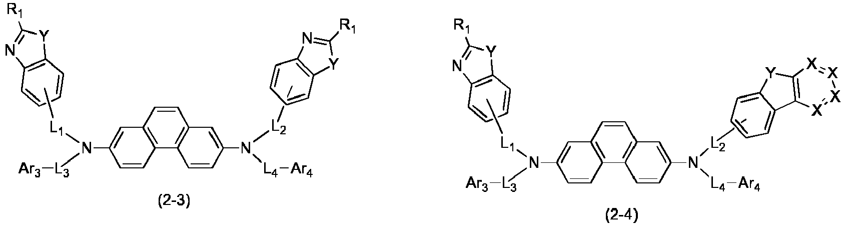

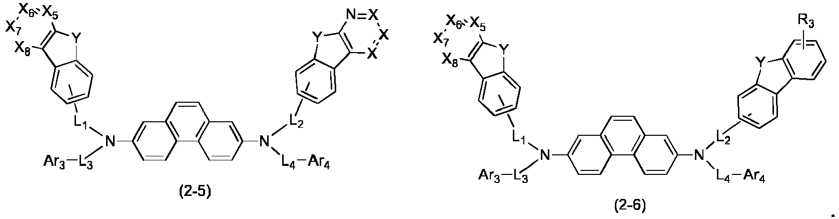

10. The mixture according to claim 8, wherein the L₁ to L₄ are each independently selected from a single bond or any one of the following groups: 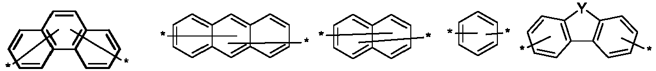 wherein: * indicates a linkage site.

11. The mixture according to claim 8, wherein the formula (1) is selected from any structure represented by formulas (3-1) to (3-6):

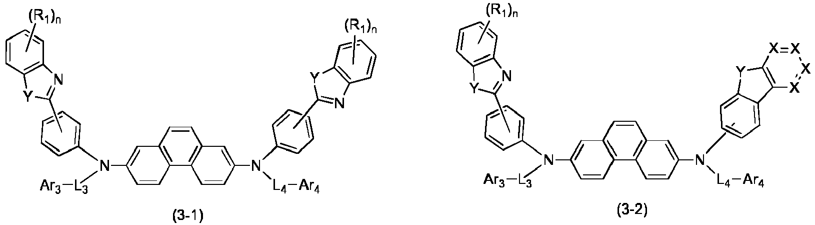

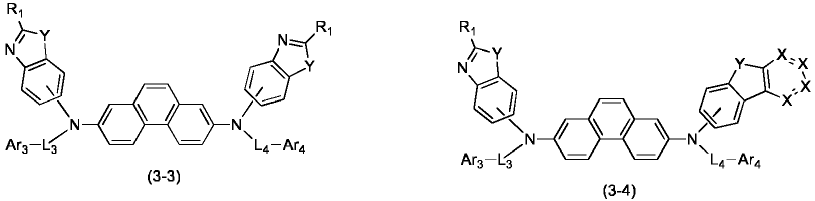

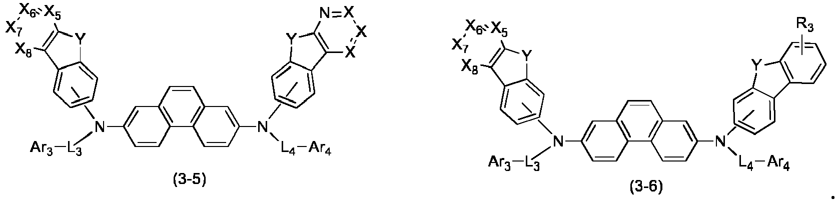

12. The mixture according to claim 8, wherein the Ar₃ and the Ar₄ are each independently selected from any group represented by formulas (B-1) to (B-7): 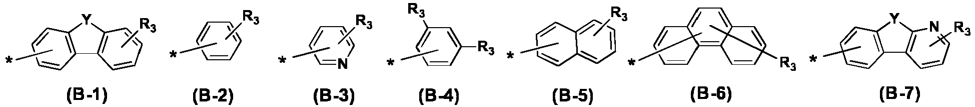 wherein:
* indicates a linkage site;
R₃ is independently selected from -H, -D, a linear alkyl group having 1 to 10 carbon atoms, a branched alkyl group having 3 to 10 carbon atoms, a cyclic alkyl group having 3 to 10 carbon atoms, a silyl group, a cyano group, an isocyano group, a hydroxyl group, a nitro group, -CF₃, -Cl, -Br, -F, -I, phenyl, biphenyl, terphenyl, or naphthyl.

13. The mixture according to claim 8, wherein *-L₃-Ar₃ and/or *-L₄-Ar₄ in the formula (1) are/is selected from the following groups: 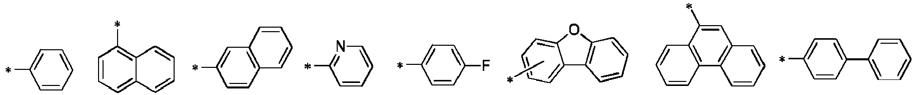 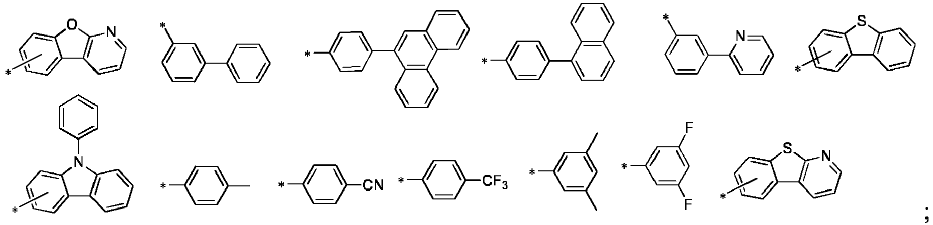 wherein: * indicates a linkage site.

14. The mixture according to claim 8, wherein the mixture is selected from any one of the following compounds:

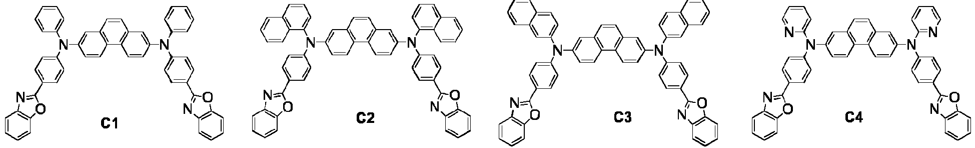

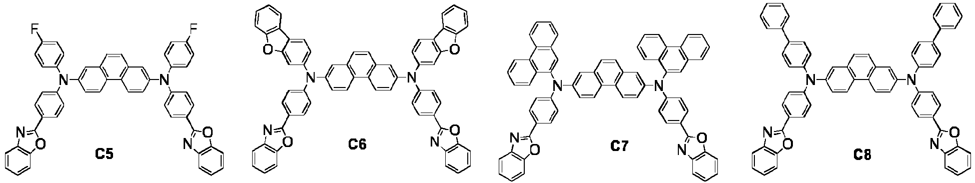

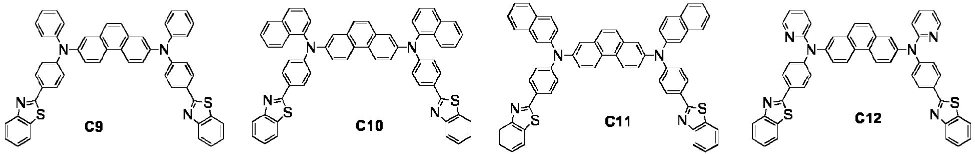

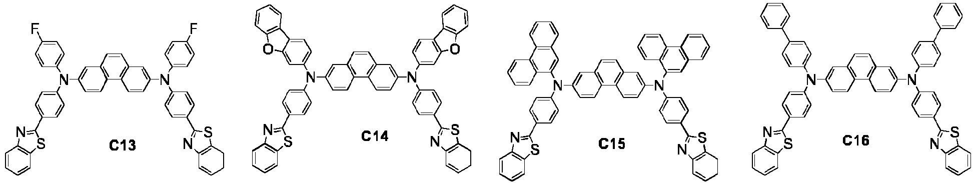

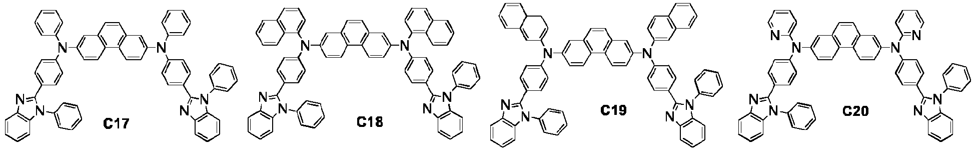

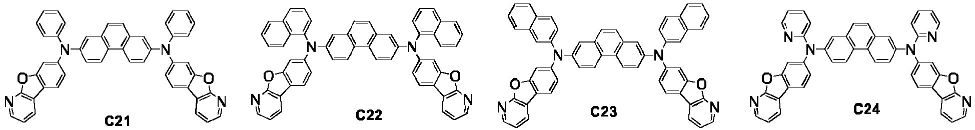

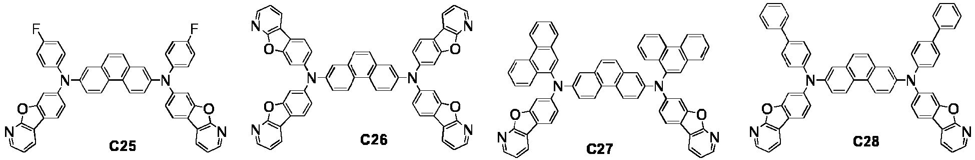

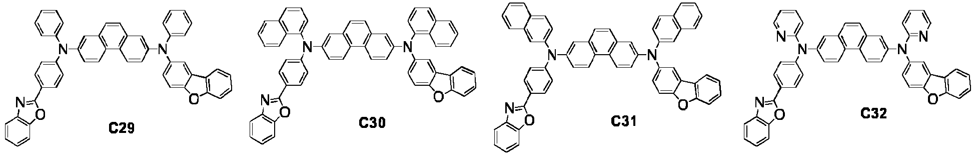

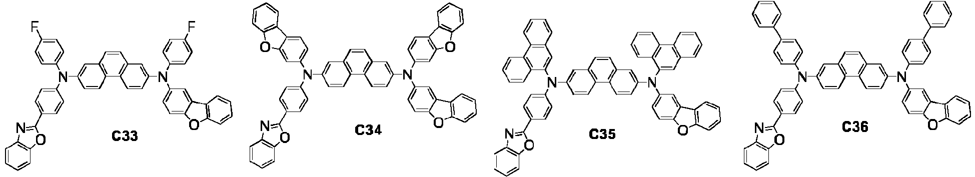

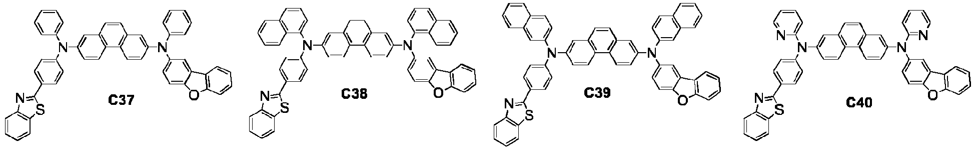

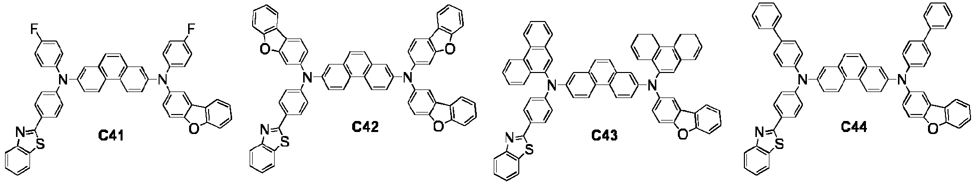

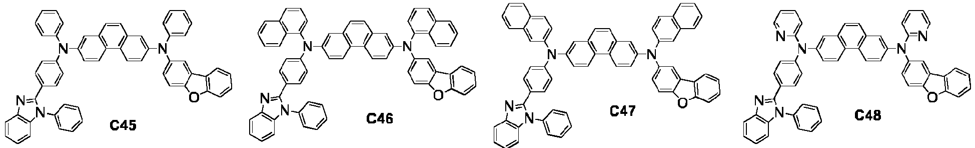

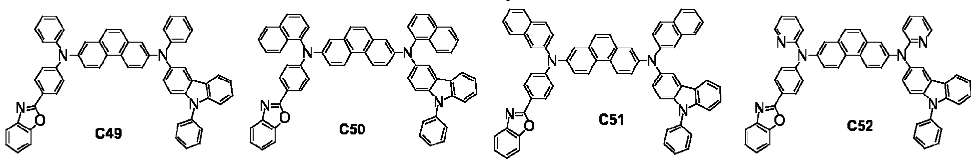

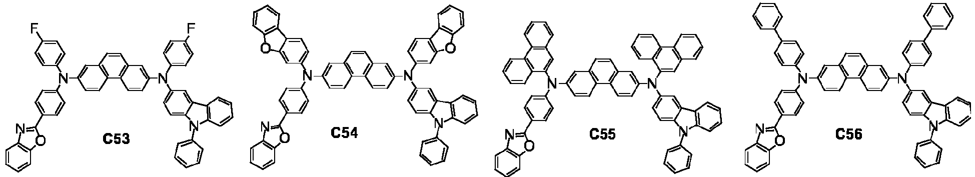

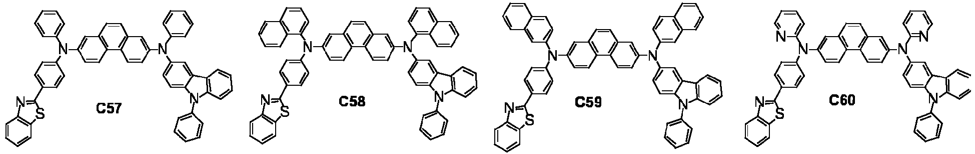

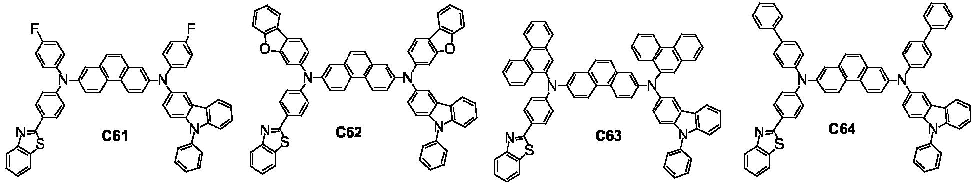

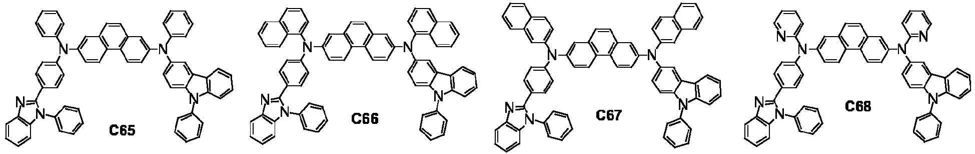

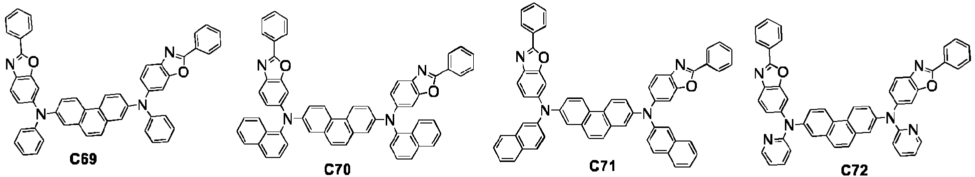

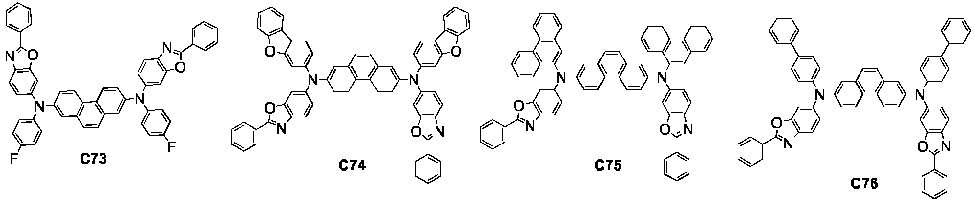

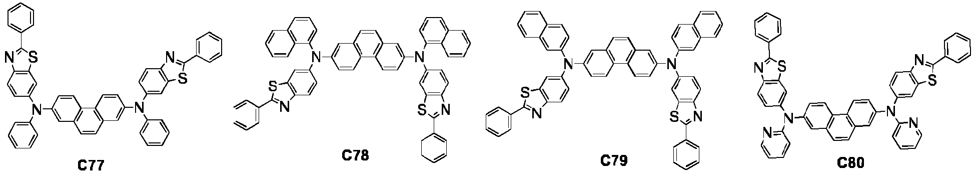

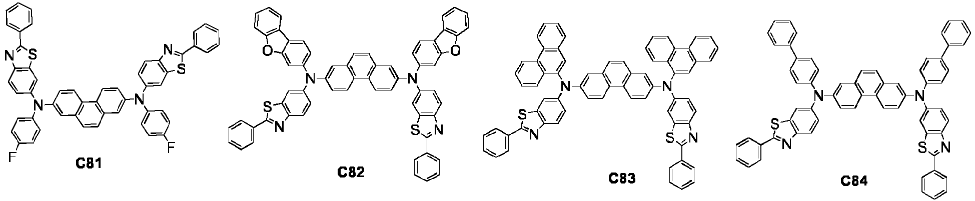

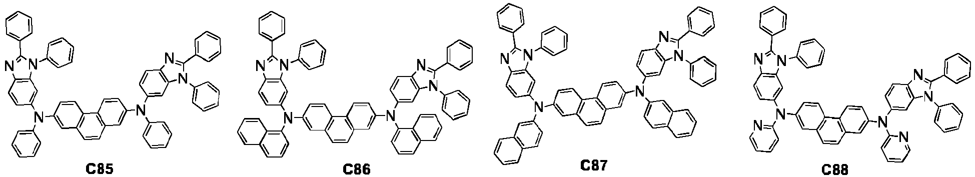

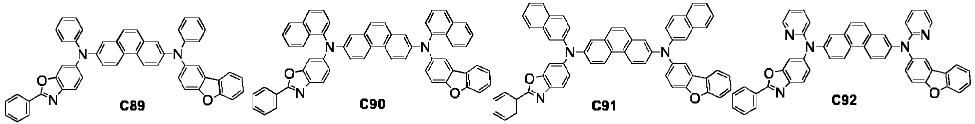

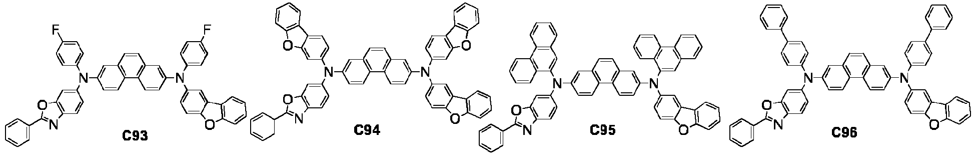

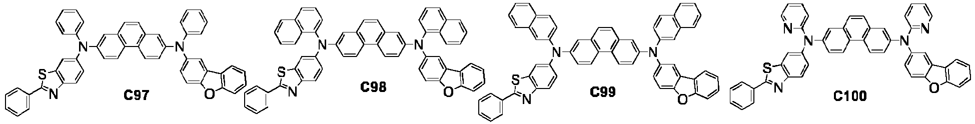

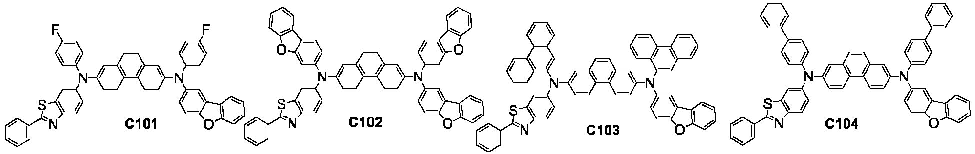

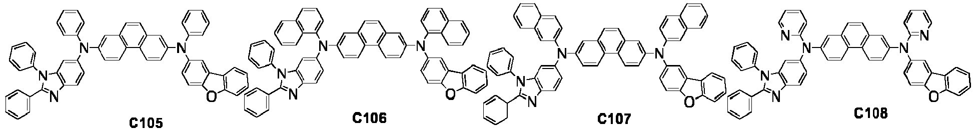

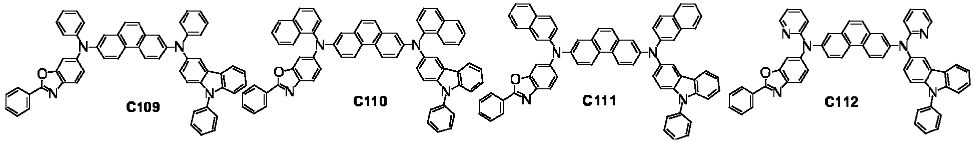

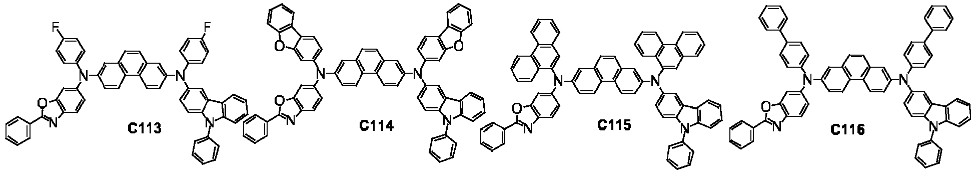

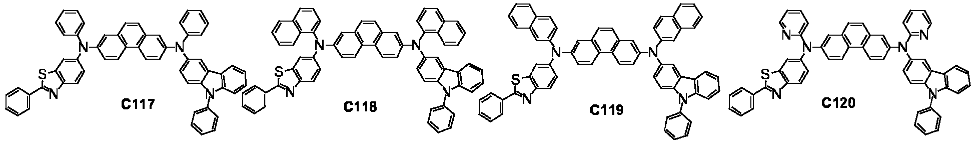

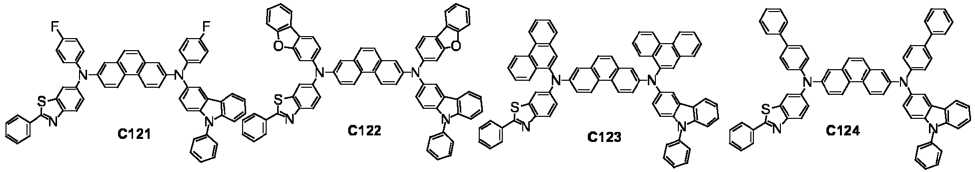

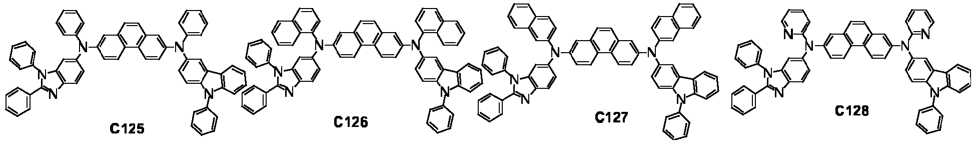

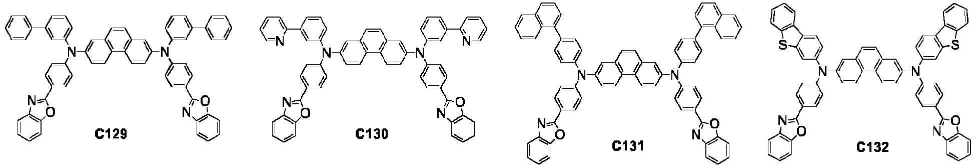

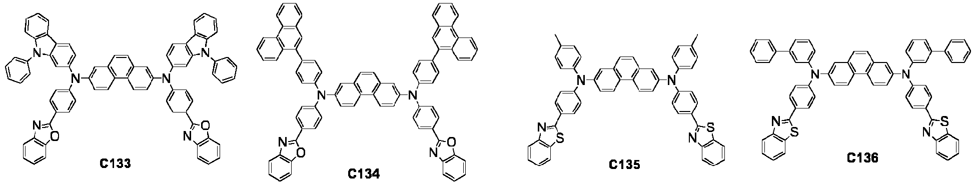

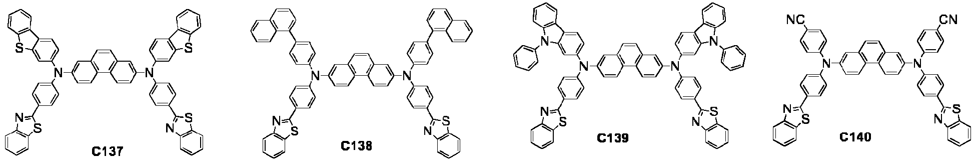

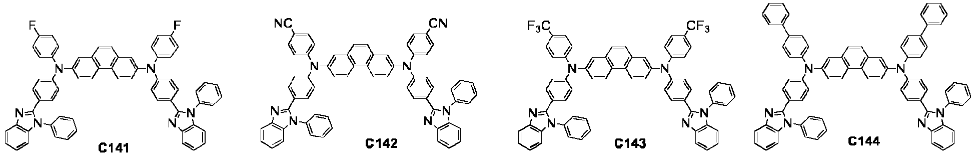

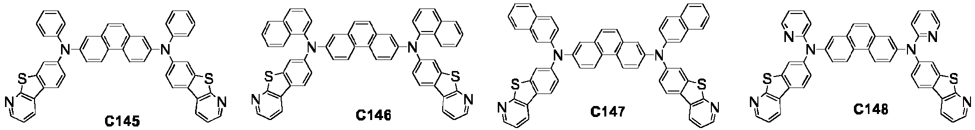

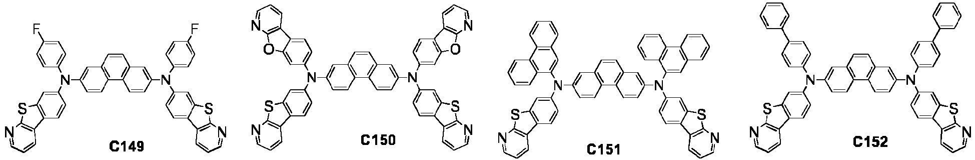

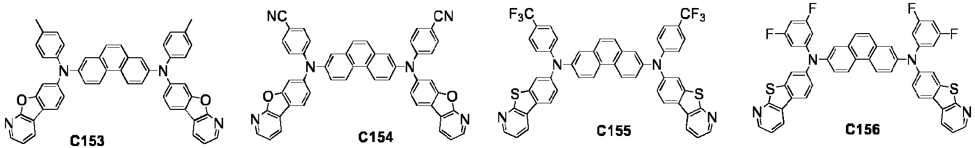

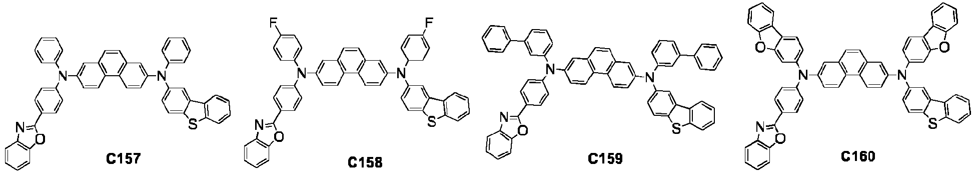

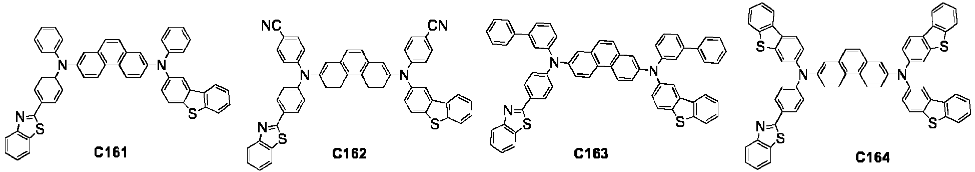

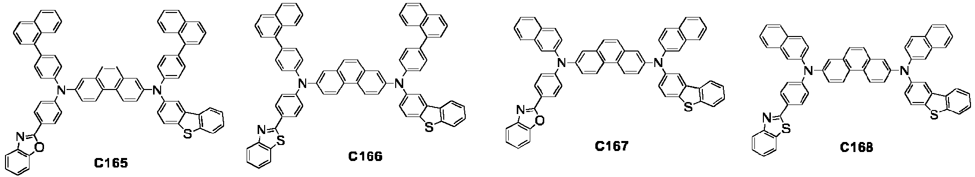

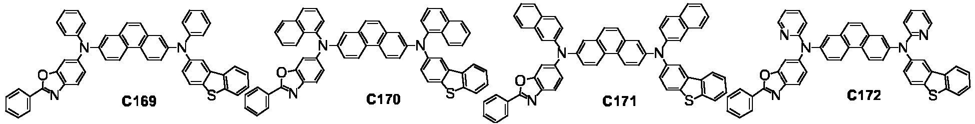

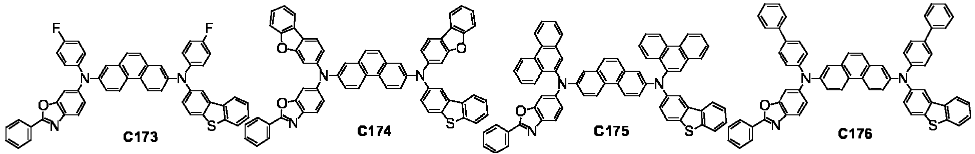

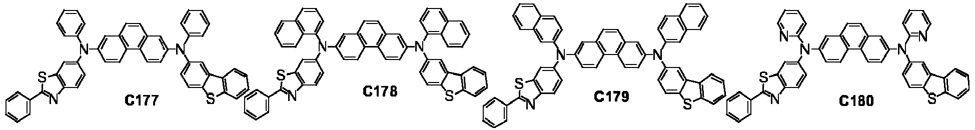

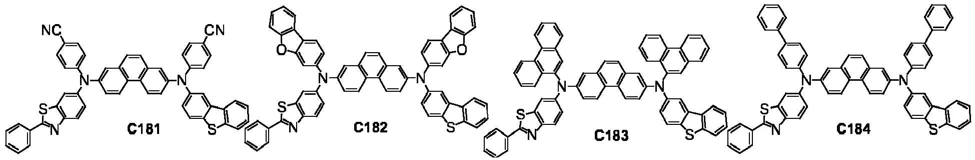

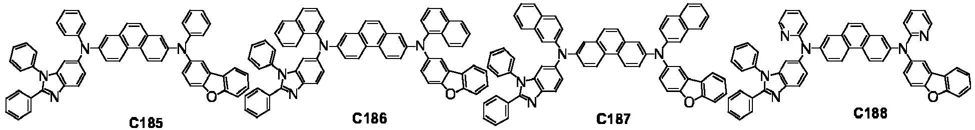

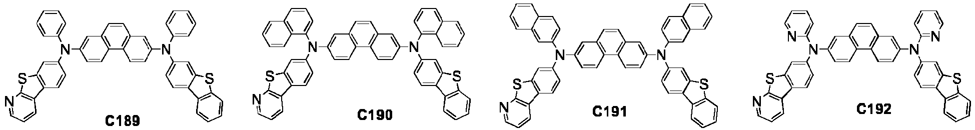

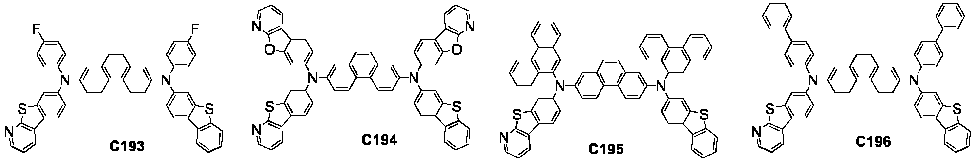

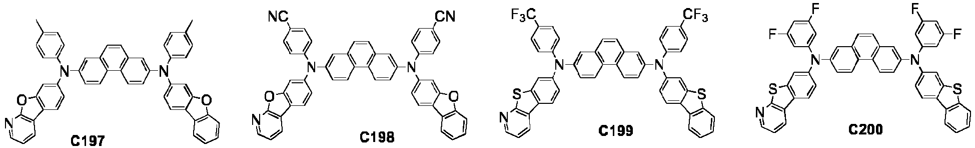

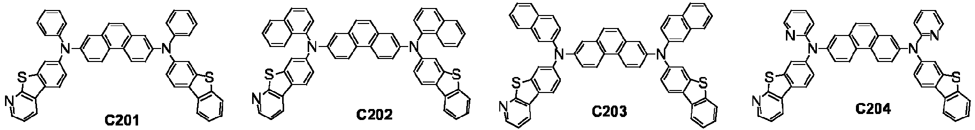

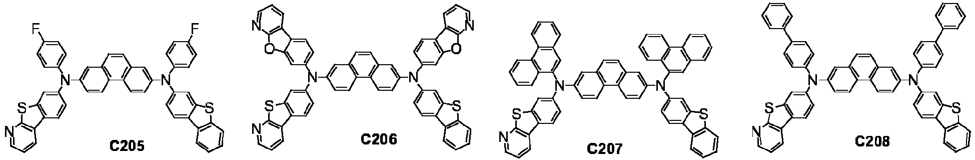

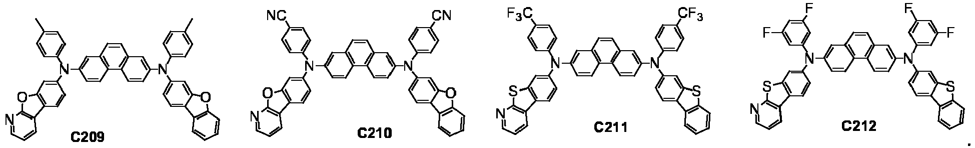

15. An organic electronic device comprising two electrodes, one or more organic functional layers disposed between the two electrodes, and a light extraction layer disposed on a surface of one of the two electrodes and on a side away from the organic functional layer, the light extraction layer comprising an arylamine organic compound, wherein the arylamine organic compound has a structure represented by formula (1): 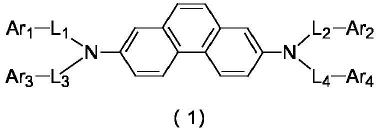 wherein:
L₁ to L₄ are each independently selected from a single bond, a substituted or unsubstituted aromatic group having 6 to 30 ring atoms, or a substituted or unsubstituted heteroaromatic group having 5 to 30 ring atoms;
Ar₁ is selected from any structure represented by formula (A-1) or formula (A-2);
Ar₂ is selected from any structure represented by formulas (A-2) to (A-6);
Ar₃ and Ar₄ are each independently selected from any structure represented by formulas (A-3) to (A-6) described above;
structures represented by formulas (A-1) to (A-6) are as follows: 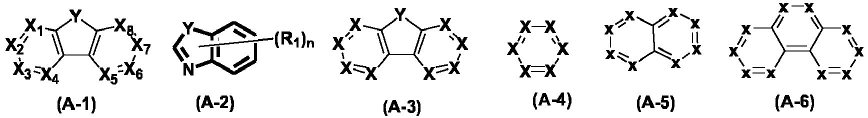
n is selected from 0, 1, 2, 3 or 4;
Y is selected from O, S or NR₂;
X and X₁ to X₈ are each independently selected from CR₃ or N, respectively, and at least one of X₁ to Xg is selected from N; and
R₁, R₂ and R₃ are each independently selected from -H, -D, a linear alkyl group having 1 to 20 carbon atoms, a linear alkoxy group having 1 to 20 carbon atoms, a branched alkyl group having 3 to 20 carbon atoms, a branched alkoxy group having 3 to 20 carbon atoms, a cyclic alkyl group having 3 to 20 carbon atoms, a cyclic alkoxy group having 3 to 20 carbon atoms, a substituted or unsubstituted silyl group, a cyano group, an isocyano group, a hydroxyl group, a nitro group, an amino group, -CF₃, -Cl, -Br, -F, -I, an alkenyl group having 2 to 20 carbon atoms, or a substituted or unsubstituted aromatic group having 6 to 20 ring atoms, or a combination thereof.

16. The organic electronic device according to claim 15, wherein the formula (1) is selected from any structure represented by formulas (2-1) to (2-6):

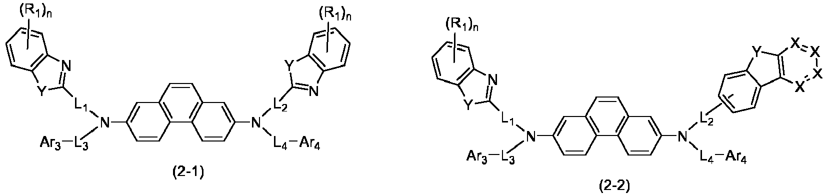

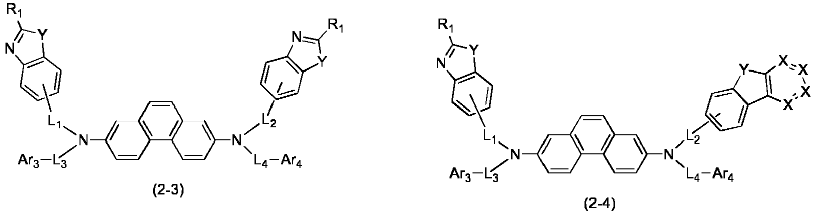

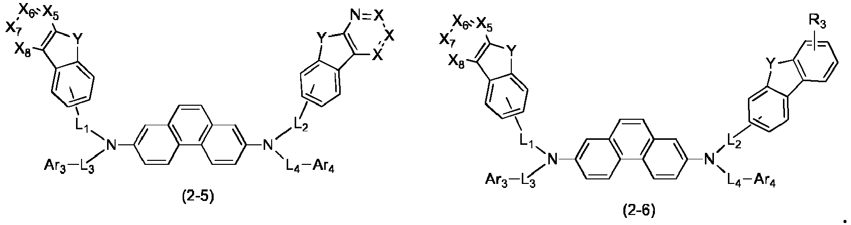

17. The organic electronic device according to claim 15, wherein the L₁ to L₄ are each independently selected from a single bond or any one of the following groups: 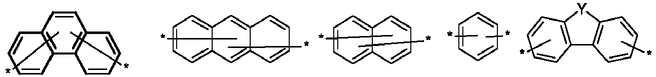 wherein: * indicates a linkage site.

18. The organic electronic device according to claim 15, wherein the formula (1) is selected from any structure represented by formulas (3-1) to (3-6):

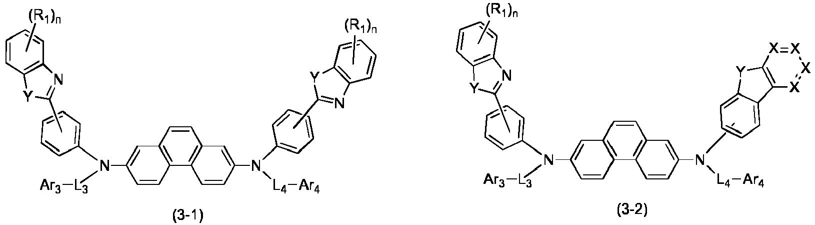

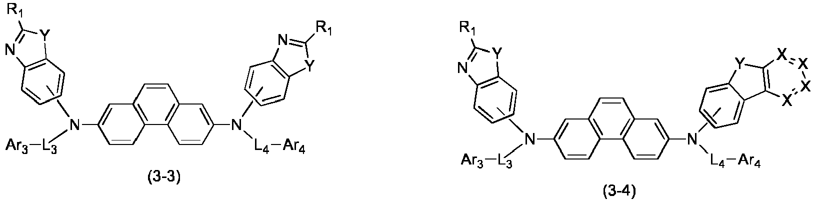

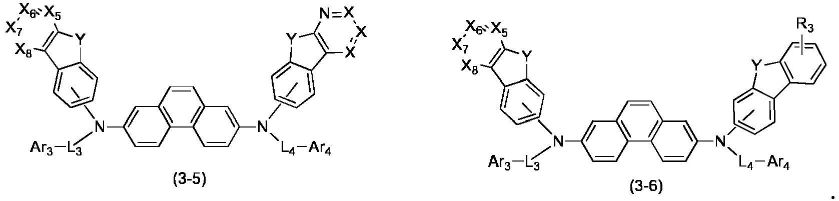

19. The organic electronic device according to claim 15, wherein the Ar₃ and the Ar₄ are each independently selected from any group represented by formulas (B-1) to (B-7): 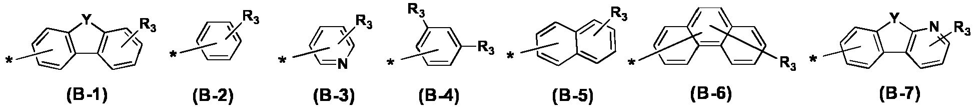 wherein:
* indicates a linkage site; and
R₃ is independently selected from -H, -D, a linear alkyl group having 1 to 10 carbon atoms, a branched alkyl group having 3 to 10 carbon atoms, a cyclic alkyl group having 3 to 10 carbon atoms, a silyl group, a cyano group, an isocyano group, a hydroxyl group, a nitro group, -CF₃, -Cl, -Br, -F, - I, phenyl, biphenyl, terphenyl, or naphthyl.

20. The organic electronic device according to claim 15, wherein *-L₃-Ar₃ and/or *-L₄-Ar₄ in the formula (1) are/is selected from the following groups: 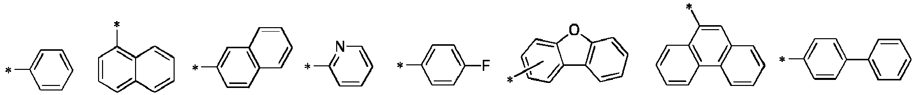 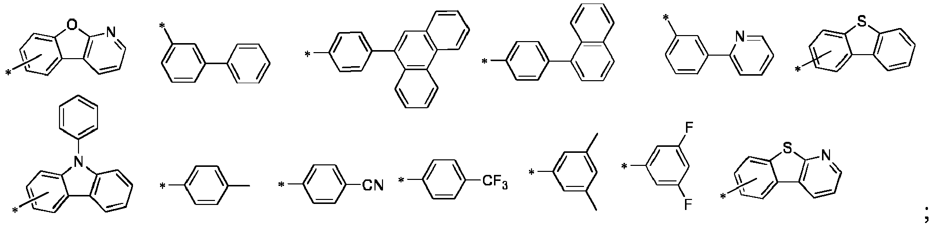 wherein: * indicates a linkage site.
